# EUROPEAN PATENT APPLICATION

(11) **EP 2 799 096 A1**
(43) Date of publication of application: **05.11.2014**
(21) Application number: 12861691.9
(22) Date of filing: 31.12.2012
(51) Int. Cl.: A61M 1/00, A61B 5/03

(54) **URINE DRAINAGE DEVICE, DRAINAGE CONTROL DEVICE, DRAINAGE CONTROL SYSTEM AND VOIDING METHOD**

(30) Priority: 31.12.2011 CN 201110457491; 31.12.2011 CN 201120571031 U
(71) Applicant: Beijing Winsunny Harmony Science and Technology Co., Ltd., Beijing 102629 (CN)
(72) Inventor: DONG, Dongsheng, Beijing 101113 (CN)
(74) Representative: Potter Clarkson LLP
(86) International application number: PCT/CN2012/087979
(87) International publication number: WO 2013/097791

(57) **Abstract**

A urine drainage device, drainage control device, drainage control system and voiding method, the urine drainage device comprising a urine inlet (31), a urine outlet (32) and a bionic bladder unit (2); the bionic bladder unit (2) comprises a main body component (21) forming a urine storage cavity (215), the urine storage cavity (215) is connected to the urine inlet (31) and the urine outlet (32) via a connecting tube (216); and further comprising a first pressure sensing surface (211) and a second pressure sensing surface (213) deforming with the change of the urine pressure in the urine storage cavity (215); a lower connecting tube (216a) is provided with a first action area (212) and a second action area (214) thereon where external forces can be applied; when external forces are applied on the two action areas (212, 214) simultaneously, the communication between the urine storage cavity (215) and the urine outlet (32) is shut off; and when any one external force applied on the action areas (212,214) is removed, the urine storage cavity (215) communicates with the urine outlet (32). The urine drainage device senses the change of the urine pressure in the urine storage cavity (215) via the deformation of the pressure sensing surfaces (211, 213), and further determines whether to conduct voiding according to the deformation level of the pressure sensing surfaces (211, 213), thus assisting a patient to void The present invention is of a simple structure and is suitable for large scale production.

## Description

This application claims the priority of China Patent Application No. 201110457491.9, filed with the Patent Office of China on December 31, 2011, titled "A URINE DRAINAGE AND CONTROL DEVICE THEREOF", and the priority of China Patent Application No. 201120571031.4, filed with the Patent Office of China on the same day, titled "A URINE DRAINAGE AND CONTROL DEVICE THEREOF", the contents of which are incorporated herein by reference in their entirety.

### FIELD OF THE INVENTION

The present invention relates to the technical field of medical device, particularly to a urine drainage device, drainage control device, drainage control system and micturition method.

### BACKGROUND OF THE INVENTION

Clinically, there are many patients of paraplegia and hemiplegia caused by cerebrovascular diseases and spinal vascular diseases, and patients with bladder fistula, coma patients, patients with high paraplegia, etc. Most of them are accompanied by disorders of micturition, and indwelling catheterization has become a common means to solve the patient's micturition dysfunction.

The urinary catheterization device used in traditional indwelling urinary catheterization consists of a urinary catheter inserted into the urethra and a urine bag connected to the lower end of the urinary catheter. The urinary catheter continuously outputs the urine in the bladder, which results in that no normal internal pressure of the bladder can be formed, so that the detrusor and internal bladder sphincter of the patient are in a long period of disuse state. Consequently, the entire urinary tract system can not be protected by using the urinary continence principle of storing urine at low pressure and micturating at low pressure. Long-term use of urinary catheters in such a structure will produce the following risks.
1. The continuous drainage by the urinary catheter makes the bladder of a patient lack filling state for a long time, easily leads to adhesions of bladder wall.
2. The continuous drainage by the urinary catheter decreases the pressure gradient between the bladder and the outside, so that, in order to ensure a smooth drainage, it is often required to increase the outer diameter, hardness, etc. of the catheter wall, which results in the oppression of the urethral mucosa of the patient and the occurrence of ischemic injury.
3. The continuous drainage by the urinary catheter makes the nerve receptors in the bladder produce adaptive changes, so that they can no longer perceive the filling of urine within the blader or complete the micturition reflex, leading to urinary retention.

Due to the above risks of a traditional urinary catheterization device, researchers of medical devices have developed a variety of medical devices for urinary catheterization, in which, by setting a preset pressure, the pressure in the bladder is collected by a pressure sensor, and when the pressure in the bladder continuously reaches or exceeds the preset pressure, it is further determined whether the duration of the pressure is equal to or greater than the preset time. If the duration of the pressure is equal to or greater than the preset time, the control component alarms to prompt micturition, or automatically starts the micturition, and automatically closes the urinary catheterization route after the completion of the urine discharge. This process simulates the normal physiological micturition of the human body, avoids the loss of bladder fuction caused by the continuous micturition in the traditional urinary catheterization manner of indwelling urinary catheterization, and achieves, to some extent, the purpose of simulating the physiological periodic micturition in human body. The purpose of the avove-mentioned setting of the preset time is to prevent automatically starting the micturition to void the bladder because of the pseudo transiently increased bladder pressure in the patient caused by postural changes, coughing, sneezing, bladder spasms and other factors, but the actual pressure has not reached the preset pressure, so that the purpose of simulating the physiological periodic micturition in human body cannot be achieved. However, the above-mentioned devices have not made the correct handling for special conditions of the pseudo transiently increased bladder pressure in the patient. Because the duration of the pseudo transiently increased bladder pressure is short and is insufficient to reach the preset time, the control component will not alarm to prompt micturition or automatically start the micturition and the urinary catheterization route is still in a closed state, so that the high pressure in the bladder will push the urine to the direction of kidney and will make the urine flow in the opposite direction, even enter into the renal pelvis, leading to bacterial infections in ureter, renal pelvis, etc. Furthermore, the high pressure in the bladder can also make the urine escape into the gap between urinary catheter and the urethra, forming overflow of urine. The urine between the urinary catherter and the urethra will become the culture medium for bacteria and viruses, and the bactera and viruses will also go backward into the bladder, leading to greater risks of infection.

In summary, for the risks of urinary reflux and overflow of urine caused by pseudo transiently increased bladder pressure as well as the contradiction between the pseudo transiently increased bladder pressure and the simulation of physiological micturition in human body, all the prior products have not disclosed any technical solutions to solve them. In practical clinical treatment, the phenomena of urinary reflux and overflow of urine always exist. The current products can no longer meet the requirements of a patient for a safe urinary catheterization. Researches of medical devices need to find a medical device capable of safely proctecting the urinary route system and achieve the simulation of the physiological micturition in human body.

Furthermore, urodynamic examination is also an item commonly used in clinical examinations. The patients underwent indwelling urinary catheterization for performing urodynamic tests need to conduct examination in s specialized testing room for urodynamic tests, wherein, firstly extracting the indwelling urinary catheter in the urethra, inserting a piezometer tube, artificially filling the bladder, conducting examination, and inserting the urinary catheter again after completing the examination. This has brought great suffering to the patient, has increased the medical burden on the patient, and has increased the risks of infection. Moreover, the existing urodynamic examinations cannot performing real-time monitoring on the urodynamic parameters, and cannot reflect the physiological state of the naturally filled bladder, and also cannot achieve individualized bionic urinary drainage, and they are especially inconvenient for those bedridden patients with indwelling urinary catheterization. Currently, no such device has been reported which is capable of performing real-time urodynamic examination on patients underwent indwelling urinary catheterization.

### SUMMARY OF THE INVENTION

The purpose of the present invention is to provide a urine drainage device comprising a urine inlet capable of connecting to the urinary catheter which is inserted into the human body and a urine outlet capable of connecting to the urine storage bag which stores urine, the urine drainge device further comprises a bionic bladder unit, the bionic bladder unit comprises a main body component capable of forming a urine storage cavity which strores urine, the urine storage cavity is connected to the urine inlet and the urine outlet via a connecting tube, the main body component comprises a first pressure transmission surface and a second pressure transmission surface capable of deforming with the change of the urine presure in the urine storage cavity, the connecting tube connecting the urine storage cavity and the urine outlet is a lower connecting tube, the lower connecting tube is provided with a first action area and a second action area on which external forces can be applied, the first action area and the second action area are oppositely disposed; when the external force is applied on both the first action area and the second action area simultaneously, the communication between the urine storage cavity and the urine outlet is blocked; the external force applied on the first action area and/or the second action area can be removed according to the deformation of the first pressure transmission surface and/or the second pressure transmission surface, and when any one external force applied on the first action area and the second action area is removed, the blockage between the urine storage cavity and the urine outlet can be removed, the communication between the urine storage cavity and the urine outlet can be restored. When the first pressure transmission surface and the second pressure transmission surface have the same function, the first pressure transmission surface or the second pressure transmission surface can be selected and used according to the actual need; similarly, when the first action area and the second action area have the same function and effect, the first action area or the second action area can be selected and used according to actual need. The urine storage cavity for storing urine is formed on the main body component, the urine storage cavity is communicated with the bladder in human body via a urinary catheter, and when the two are in different horizontal positions, the urine pressure in the bladder in human body and the urine pressure in the urine storage cavity can be mutually calculated according to the height difference, and therefore, collecting the changes of the urine pressure in the urine storage cavity can directly obtain the changes of urine pressure in the bladder in human body.

The change of the urine pressure in the urine storage cavity of the urine drainage device can be reflected by the deformation of the pressure transmission surfaces, and in turn it is further determined whether to conduct micturition according to the deformation level of the pressure transmission surfaces, thereby assisting the function of a patient to micturate. When controlled in an artificial manner, micturition can be conducted manually for the patient according to the deformation level and the micturition desire of the patient, which is high in flexibility; when mechanically controlled and electrically controlled, the examples described below can be referred for understanding. Furthermore, the urine drainage device in such a structure is of a simple structure and is suitable for large sclae production.

Preferably, the first action area and the second action area are oppositely disposed, and form a lower connecting tube when they are fastened. Removal of the external force acted on any one of the action areas can form the sectional area required for the communication between the urine storage cavity and the urine outlet. Of course, when all the external forces acted on both the first action area and the second action area are removed, the communication between the urine storage cavity and the urine outlet will become smoother.

Preferably, the main body component comprises two diaphragms, one diaphragm forms the first pressure transmission surface and the first action area, and the other diaphragm forms the second pressure transmission surface and the second action area. The two diaphragms are fastened and sealed, and the internal cavity formed by the oppositely disposed first pressure transmission surface and second pressure transmission surface is the urine storage cavity.

Preferably, the first pressure transmission surface and the second transmitting surface are made of an elastic material, and the first action area and the second action area are made of a flexible material. Of couse, the first pressure transmission surface, the second transmitting surface, the first action area and the second action area can be made of one flexible material having elasticity.

Preferably, the bionic bladder unit further comprises a hard cover housing. After fastening, the cover housing is capable of covering the portions on the main body component except the first pressure transmission surface, the second transmitting surface, the first action area and the second action area. The cover housing can protect the above-mensioned first pressure transmission surface, first action area, second transmitting surface and second action area against damage from exteral forces, and can fix the position of the entire bionic bladder unit.

Preferably, the urine inlet and the urine outlet are connected to a urine inlet tube and a urine outlet tube respectively, and the urine inlet tube is provided with a urinary catheter connector for connecting to the urinary catheter. The urinary catheter connector and/or the urine inlet tube are provided with a self-sealable region for temperature collecting and/or urine sampling. The self-sealable region for collecting temperature can be penetrated by a sharp temperature-collecting device, and can self-seal after extracting the collecting device, so that the urine does not leak out and contaminate the medical staff. The self-sealable region for urine sampling also has the above-mentioned self-sealable function.

The present invention further privides a urine drainage control device, comprising a urine drainage portion, a control portion and a housing containing the urine drainage portion and the control portion, the urine drainage portion is the above-mentioned urine drainage device; the control portion comprises a pressure transmission component and a closure component, the pressure transmission component is capable of contacting with the deformed first pressure transmission surface and/or second pressure transmission surface, and shifts in position by the action of force produced by the deformation of the first pressure transmission surface and/or the second pressue transmitting surface; the closure component is capable of shifting along with the shift in position of the pressure transmission component, and when shifting in position, the closure component is capable of applying or removing the external force acted on the first action area and/or the second action area, thereby blocking or unblocking the communication between the urine storage cavity and the urine outlet.

Preferably, there is a gap between the pressure transmission component and the corresponding pressure transmission surface thereof, and when the pressure transmission surface deforms along with the increasing pressure in the urine storage cavity, the gap can be tapered until eliminated. The purpose to leave the gap is to ensure that the urine storage cavity has a certain volume, so that the pressure transmission surface is capable of deforming within a resonable space, and simultaneously prevents the adhesion between the diaphragms. The pressure transmission surface corresponding to the pressure transmission component refers to a pressure transmission surface capable of having mechanical relationship with the pressure transmission component, and can be the first pressure transmission surface, or can also be the second pressure transmission surface. Of course, the corresponding action area can also be the first action aera or the second action area.

Preferably, the control portion further comprises a resistance component, the resistance component applies a predetermined resistance to the pressure transmission component and/or the closure component to block the deformation of the corresponding pressure transmission surface, and when the force of the corresponding pressure transmission surface acted on the pressure transmission component and/or the closure component is greater than the predetermined resistance, the pressure transmission component drives the closure component to shift in position. The resistance of the resistance component is produced by a spring, one end of the spring connects to the housing, and the other end connects to the pressure transmission component or the closure component.

Preferably, the resistance component further comprises an adjustment portion which adjusts the resistance component, the adjustment portion can compress or release the spring of the above-mentioned resistance component, so as to adjust the predetermined resistance applied by the spring to the pressure transmission component or the closure component.

Preferably, the pressure transmission component and the closure component are a unitary structure, the control portion further comprises a rotation axis, and when the pressure transmission component shifts in position along with the deformation of the corresponding pressure transmission surface, the pressure transmission component and the closure component rotate together around the rotation axis, so that the closure component applies or removes the external force acted on the corresponding action area, thereby blocking or unblocking the communication between the urine storage cavity and the urine outlet.

Preferably, the pressure transmission component and the closure component are a unitary structure, the pressure transmission component is provided with a guide rod, the housing is provided with a socket, the guide rod is capable of shift in the socket in the stretching direction of the spring, and when the pressure transmission component shifts in position along with the deformation of the corresponding pressure transmission surface, the pressure transmission component and the closure component shift together in the socket, so that the closure component applies or removes the external force acted on the corresponding action area, thereby blocking or unblocking the communication between the urine storage cavity and the urine outlet.

Preferably, the pressure transmission component and the closure component are a split design, the pressure transmission component comprises a first gear, the closure component comprises a closure head and a second gear engaged with the first gear, both the first gear and the second gear are fixed with respect to the housing; the spring and the pressure transmission component are connected by a movable axis, and when the pressure transmission component shifts in position along with the deformation of the corresponding pressure transmission surface, the first gear drives the rotation of the second gear so as to drive the closure head to apply or remove the external force acted on the corresponding action area, thereby blocking or unblocking the communication between the urine storage cavity and the urine outlet.

Preferably, the pressure transmission component and the closure component are hinged, and when the pressure transmission component shifts in position along with the deformation of the corresponding pressure transmission surface, it drives the rotation of the closure component, applies or removes the external force acted on the corresponding action area, thereby blocking or unblocking the communication between the urine storage cavity and the urine outlet.

Preferably, the control portion comprises a manually controlled valve, so as to manually apply or remove the external force acted on the corresponding action area according to the parameters such as the deformation level, thereby blocking or unblocking the communication between the urine storage cavity and the urine outlet.

The urine drainage control device employs mechanical principles, transfers the pressure produced by the deformation of the pressure transmission surface to the pressure transmission component, and thus the pressure transmission component drives the closure component to apply or remove the external force on the corresponding action area, thereby blocks or unblocks the communication between the urine storage cavity and the urine outlet. When the device is initially used, the closure component applies a certain external force acted on the corresponding action area, and blocks the communication between the urine storage cavity and the urine outlet, and when the pressure in the bladder reach the threshold, the force produced by the deformation of the pressure transmission surface is capable of triggering the shift in position of the pressure transmission component and the closure component, thereby removes the external force acted on the action area, the urine storage cavity and the urine outlet communicate, the urine enters into a urine storage bag, so as to complete the process of releasing pressure and micturition. The advantageous effect of the device is that, when the patient coughs, turns over or the like so as to cause a pseudo instant increase of the bladder pressure, the device is capable of instantly releasing the pressure, so as to prevent the risks of urine reflux, overflow of urine, etc. caused by the instantly increase of the pressure in the bladder. The urine drainage device can also simulate the normal physiological micturition of the human body in coorperation with the manually controlled valve. When the patient feels a micturition desire or the deformation level of the pressure transmission surface is relatively greater, the patient can manually start the manually controlled valve to perform micturition. This helps to train the bladder function of the patient.

The present invention further provides a urine drainage control system, comprising a urine drainage control portion and an electric control portion, the urine drainage control portion and the electric control portion are contained in the housing, the urine drainage control portion is the above-mensioned urine drainage control device, the electric control portion comprises a central controller, an execution component, and a pressure sensor that collects the pressure in the bladder, the central controller receives the pressure signal outputted by the pressure sensor, and when the pressure is equal to or greater than the micturition threshold, and the duration is equal to or greater than the preset judgement time, the central controller outputs an instruction of communicating the urine storage cavity and the urine outlet to the execution componet.

The urine drainage control system sufficiently contemplates the special conditions of a pseudo transiently increased bladder pressure in the patient caused by postural changes, coughing, sneezing, bladder spasms and other factors, and at the same time contemplates the condition of normal physiological micturition when the bladder of the patient reach an effective pressure, which provides a "double insurance" for the process of micturition, not only achieves the function of training the bladder of the patient, but also avoids the the risks of urine reflx, overflow of urine, etc. When the bladder pressure of the patient is equal to or greater than the preset micturition threshold, and the duration is equal to or greater than the judgement time, the electric control portion controls the communication between the urine storage cavity and the urine outlet, and this process simulates the process of normal physiological micturition of the human body, and helps to restore the bladder function of the patient; when the special conditions of a pseudo transiently increased bladder pressure in the patient occur, the risks of urinary reflux and overflow of urine in the patient are avoided because the function of releasing pressure and micturition are started by the mechanical portion. One aspect of the purpose of the above setting avoids the defects that a general electric control device cannot solve the risk caused by pseudo transiently increased bladder pressure in the patient, and the other aspect simulates the normal physiological micturition more precisely. For a common electric control device, when the pseudo transiently increased bladder pressure in the patient occurs, if the micturition is started, this is equivalent to empty the urine in the bladder of the patient before the actual pressure arrives the required micturition pressure, and thus cannot truly simulate the process of normal physiological micturition, if the micturition is not started, the risks of overflow of urine and urine reflux brought by the transiently increased pressure cannot be solved; after adding the mechanical device, due to the chararistics of a mechanical structure, the transiently increased pressure can drive the pressure transmission component and the closure component to shift in position and thus communicate the urine storage cavity and the urine outlet, and with the rapid decrease of pressure, the deformation level of the pressure transmission surface decrease, and the amount of shift of the pressure transmission component and the closure component also decrease, the closure component again applies external force on the corresponding action area and blocks the communication between the urine storage cavity and the urine outlet, so as to effectively avoid the risks of overflow of urine and urine reflux.

Preferably, the pressure sensor collects the blader pressue transmitted by the corresponding pressure transmission surface, and the execution component applies or removes the external force on the corresponding action aera, thereby blocking or unblocking the communication between the urine storage cavity and urine outlet. The pressure transmission surface corresponding to the pressure sensor refers to a pressue transmitting surface capable of transmitting the bladder pressure to the pressure sensor, and can be the first pressure transmission surface, or can be the second pressure transmission surface. Of course, the corresponding action area can also be the first action area or the second action area.

Preferably, the driving mechanism of the execution component is an electric motor, a solenoid valve, an air pump, etc. When utilizing an air pump as the execution component, the execution component further comprises an air sac, the central controller controls the air pump to inflate the air sac according to the pressure signal from the pressure ssensor in order that the air sac applies external force acted on the corresponding action area, or controls the air sac to deflate in order to remove the external force acted on the corresponding action area, thereby blocking or unblocking the communication between the urine storage cavity and the urine outlet.

Preferably, the drainage control device is provided with a positioning slot, a housing covering the urine drainage control system is provided with a positioning projection fitted to the positioning slot, so that it is more convenient and efficient when using the device.

Preferably, the urine drainage control system further comprises a weight sensor, which is used for real-time monitoring the weight of urine in the urine storage bag, and outputs the signal to the central controller.

Preferably, the urine drainage control system further comprises a display component, an input component, a data storage component; the display component connects to the central controllor, for displaying the monitored data and the artificially set data as well as the calculated result by the central controller; the input component connects to the central controller, for setting parameters and/or querying the storage record of the data storage component and the calculated result of the central controller; the data strorage component which is used for the output of data and the input of data, the data storage component is capable of storing the parameter set by the input component, as well as the urine flow rate curve, the urinary output curve, the bladder pressure curve, the abdominal pressure curve, etc. that are anlyzed and generated by the central controller according to the parameters of pressure, weight, time, urine density, etc., and transports the above-mentioned curves to an external device, and is capable of further tranporting the parameter recorded on the external devices to the central controller;

the pressure sensor of the urine drainage system can real-time monitor the bladder pressure, and transport the pressure signal to the central controller, a series of curves and parameters reflecting the bladder function can be obtained after processing by the central controller, including monitoring the residual urine volume in the bladder, bladder volume, bladder volume when initially feeling the sense of urine, the contraction force of detrusor during micturition, etc. The weight sensor can real-time monitor the change of urine weight in the urine storage bage, the change of urine amount over time can be obtained after the weight signal is transported to the central controller and processed, so as to obtain the flow rate curves and the micturition curves. The weight sensor and the central controller may further precisely meter the urine amount over 24 h, the urine amount per hour, etc., and display whether the human body is in an abnormal condition of polyuria, oliguria, anuria, oliguria over 1 h, etc. on the display component, according to the preset analytical value of urine amount. The urine drainage control system can perform urodynamic tests, which is especially suitable for the long-term bedridden patients patients with indwelling urinary catheterization, and can perform 24 h real-time urodynamic monitoring on a patient in bed, and can timely, accurately, dynamically obtain the urodynamic parameters; there is no need to repeatedly insert and extract the urinary catheter, the bladder is naturally filled, the agony of the patient is reduced, the risk for infection is decreased, and the naturally filled physiological state of bladder can be truly reflected.

In order to more accurately reflect the bladder pressure and perform urodynamic test, the present device is further provided with a mobile display component and an external abdominal pressure acquisition component, the external abdominal pressure acquisition component can collect the abdominal pressure of human body, and transport the collected signal to the central controller, the central controller is provided with a connector which connect to the external abdominal pressure acquisition component, the mobile display component is wirelessly connected to the central controllor, for displaying the contents displayed by the external abdominal pressure acquisition component.

The purpose of the present invention is to further provide a micturition method which protects the bladder function, the method comprises:
presetting a threshold of releasing pressure as well as a time to start releasing pressure and a duration of releasing pressure;
collecting the pressure in the bladder;
judging whether the pressure is equal to or greater than the preset threshold of releasing pressure;
if the judgement result is that the pressure is equal to or greater than the preset threshold of releasing pressure, then starting micturition to release pressure within the time to start releasing pressure, and the time for releasing pressure is the preset duration of releasing pressure.

Preferably, the method further comprises: presetting a micturition threshold, a judgement time and a micturition duration, the micturition threshold is smaller than the above-mentioned threshold of releasing pressure; collecting the pressure in the bladder; judging whether the pressure is equal to or greater than the preset threshold of releasing pressure; if the judgement result is that the pressure is equal to or greater than the preset threshold of releasing pressure, then stating micturition to release pressure within the time to start releasing pressure, the time for releasing pressure is the preset duration of releasing pressure; if the judgement result is that the pressure is smaller than the preset threshold of releasing pressure, then continuing to judge whether the pressure is equal to or greater than the micturition threshold, if the judgement result is that the pressure is equal to or greater than the preset micturition threshold, then further judging whether the duration of the pressure is equal to or greater than the judgement time, if the judgement result is that the duration of the pressure is equal to or greater than the judgement time, then performing micturition, and the time for micturition is the preset micturition duration.

Of course, it can also be judged whether the pressure is equal to or greater than the preset micturition threshold; if the judgement result is that the pressure is equal to or greater than the preset micturition threshold, then further judging whether the pressure is equal to or greater than the preset threshold of releasing pressure, if the judgement result is that the pressrue is equal to or greater than the preset threshold of releasing pressure, then starting the micturition to release pressure within the time to start releasing pressure, the time for releasing pressure is the preset duration of releasing pressure; if the judgement result is that the pressure is equal to or greater than the preset micturition threshold and smaller than the micturition threshold, then continuing to judge whether the duration of the pressure is equal to or greater than the judgement time, if the judgement result is that the duration of the pressure is equal to or greater than the judgement time, then performing micturition, and the time for micturition is the preset micturition duration; if the judgement result is that the pressure is smaller than the preset micturition threshold, then continuing to collect the pressure in the bladder for performing judgement.

Preferably, the duration of releasing pressure is smaller than the micturition duration. The time to start releasing pressure is a shorter time, the time to start releasing pressure is smaller than the judgement time, and may be close to zero in some conditions, that is to immediately perform micturition to release pressure. Of course, the above-mentioned process is repeated, and when all the judgement is negative, the judgement for pressure is continued to perform.

The purpose of the present invention is to further proved a urine drainage control system, comprising a urine inlet capable of connecting to a urinary catheter which is inserted into the human body, a urine outlet capable of connecting to a urine storage bag which stores the urine, and at least a pressure transmission surface on at least one area between the urine inlet and the urine outlet, the pressure transmission surface is capable of transmitting the pressure in the bladder, the urine drainage control system further comprises:
a pressure sensor, which collects the pressure in the bladder through the above-mentioned pressure transmission surface, and generates and transports pressure signal, the pressure transmission surface is not necessarily deformed, as long as the pressure can be collected by the pressure sensor;
a central contoller, which receives the pressure signal outputted by the pressure sensor, and judges whether the pressure is equal to or greater than the threshold of releasing pressure, if the judgement result is yes, then starting the micturition to release pressure within the time to start releasing pressure, the time for releasing pressure is the preset duration of releasing pressure, if the judgement result is no, then continuing to judge whether the pressure is equal to or greater than the micturition threshold, if the judgement result is yes, then further judging whether the duration of the pressure is equal to or greater than the judgement time, if the judgement result is yes, then outputing an instruction of openning the urine outlet to perform micturition, and the time for micturition is the preset micturition duration;
or central controller recives the pressure signal outputted by the pressure sensor, and judges whether the pressure is equal to or greater than the preset micturition threshold, if the judgement result is yes, then further judging whether the pressure is equal to or greater than the preset threshold of releasing pressure, if the judgement result is yes, then starting the micturition to release pressrue within the time to start releasing pressure; if the judgement result is no, then continuing judging whether the duration of the pressure is equal to or greater than the judgement time, if the judgement result is yes, then outputing an instruction of opening the urine outlet to perform micturition, and the time for micturition is the preset micturition duration;
an execution component, which receives the instruction from the central controller, and controls whether the urine flows out from the urine outlet.

The above-mentioned micturition method is intended to combine the pseudo transiently increased bladder pressure of the patient with simulating the normal physiological micturition in human body, and conducting pressure collecting and communication control all by utilizing electronically controlled manner, which has the following advantages as compared to a mechanical structure: more precise, the product is smaller in volume, and the operation is more convenient, etc.

Preferably, the driving mechanism of the execution component is an electric motor, a solenoid valve, an air pump, etc. When selecting an air pump as the execution component, the execution component further comprises an air sac, the central controller controls the air pump to inflate the air sac according to the pressure signal from the pressure ssensor in order that the air sac applies external force acted on the corresponding action area, thereby closing the urine outlet; or controls the air sac to deflate in order to remove the external force acted on the corresponding action area, thereby opening the urine outlet.

Preferably, the urine drainage control system further comprises a weight sensor, the weight sensor is used for real-time monitoring the weight of urine in the urine storage bag, and transports the weight signal to the central controller.

Preferably, the urine drainage control system further comprise a display component, an input component, a data storage component; the display component connects to the central controllor, for displaying the monitored data and the artificially set data and the calculated result by the central controller; the input component connects to the central controller, for setting parameters and/or querying the storage record of the data storage component and the calculated result by the central controller; the data strorage component which is used for the output of data and the input of data is capable of storing the parameters set by the input component, as well as the urine flow rate curve, the urinary output curve, the bladder pressure curve and the abdominal pressure curve that are anlyzed and generated by the central controller, and is capable of transporting the above-mentioned curves to an external device, and is capable of further tranporting the parameter recorded on the external devices to the central controller;

In order to more accurately reflect the bladder pressure and perform urodynamic test, the above-mentioned system further comprises a mobile display component and an external abdominal pressure acquisition component, the external abdominal pressure acquisition component can collect the abdominal pressure of human body, and transports the collected signals to the central controller, the central controller is provided with a connector which connects to the external abdominal pressure acquisition component; the mobile display component is wirelessly connected to the central controllor, for displaying the contents displayed by the external abdominal pressure acquisition component.

Preferably, the pressure transmission surface is made of an elastic material, and can deform along with the change of pressure in the bladder. The urine drainage control system further comprises a control portion, the control portion comrpises a pressure transmission component and a closure component, the pressure transmission component is capable of contacting with the pressure transmission surface, and shifts in position by the action of force produced by the deformation of the pressure transmission surface; the closure component can shift along with the shift in position of the pressure transmission component, and when shifting in position, the closure component is capable of controling whether the urine flows out from the urine outlet.

### DESCRIPTION OF THE DRAWINGS

Figure 1 is the overall half-sectional schematic view of the bionic bladder unit of the urine drainage device accroding to the present invention.
Figure 2 is the partially enlarged half-sectional schematic view of A in figure 1.
Figure 3 is the exploded schematic view 1 of the bionic bladder unit of the urine drainage device accroding to the present invention.
Figure 4 is the exploded schematic view 2 of the bionic bladder unit of the urine drainage device accroding to the present invention.
Figure 5 is the perspective schematic view 1 of the bionic bladder unit of the urine drainage device accroding to the present invention.
Figure 6 is the perspective schematic view 2 of the bionic bladder unit of the urine drainage device accroding to the present invention.
Figure 7 is the structural schematic view of the connection between the urine drainage device according to the present invention and the urine storage bag.
Figure 8 is the partially enlarged schematic view of B in figure 7.
Figure 9 is the overall half-sectional schematic view of the urine drainage control device according to the present invention.
Figure 10 is the exploded perspective schematic view of the urine drainage control device.
Figure 11 is the exploded perspective schematic view of the resistance component of the urine drainage control device.
Figure 12 is the half-sectional schematic view of the manually controlled valve of the urine drainage control device in open state.
Figure 13 is the half-sectional schematic view of the manually controlled valve of the urine drainage control device in closed state.
Figure 14 is the structural schematic view 1-1 of the urine drainage control device accroding to the present invention in closed state.
Figure 15 is the structural schematic view 1-2 of the urine drainage control device accroding to the present invention in open state.
Figure 16 is the structural schematic view 2-1 of the urine drainage control device accroding to the present invention in closed state.
Figure 17 is the structural schematic view 2-2 of the urine drainage control device accroding to the present invention in open state.
Figure 18 is the structural schematic view 3-1 of the urine drainage control device accroding to the present invention in closed state.
Figure 19 is the structural schematic view 3-3 of the urine drainage control device accroding to the present invention in open state.
Figure 20 is the structural schematic view 4-1 of the urine drainage control device accroding to the present invention in closed state.
Figure 21 is the structural schematic view 4-2 of the urine drainage control device accroding to the present invention in open state.
Figure 22 is the structural schematic view of the urine drainage control system according to the present invention.
Figure 23 is the flow chart of the micturition method of the urine drainage control system accroding to the present invention.
Figure 24 is the flow chart 1 of the micturition method which protects the bladder function according to the present invention.
Figure 25 is the flow chart 2 of the micturition method which protects the bladder function according to the present invention.
Figure 26 is the structural schematic view of the urine drainage control system of the micturition method prepresented by the example figure 24 and figure 25.

pressure transmission component 11, first gear 111, closure component 12, closure head 121, second gear 122, resistance component 13, spring 131, adjustment portion 132, handle 132a, index plate 132b, right threaded housing 132c, left threaded housing 132d, limit component 14, rotation axis 15, bionic bladder unit 2, main body component 21, first diaphragm 21a, second diaphragm 21b, first pressure transmission surface 211, first action area 212, second pressure transmission surface 213, second action area 214, urine storage cavity 215, connecting tube 216, lower connecting tube 216a, cover housing 22, right cover housing 222, left cover housing 221, bare hole 223, urine inlet 31, urine outlet 32, urinary inlet tube 41, urinary outlet tube 42, urine storage bag 43, hook 431, urinary catheter connector 44, temperature measuring 45, urine sampling 46, pressure sensor 51, central controller 52, execution component 53, weight sensor 54, display component 55, input component 56, data storage component 57, mobile display component 58, external abdominal pressure acquisition component 59, housing 6, manually controlled valve 7, rotation handle 71, valve main body 72, reset component 73, valve spool 74, valve housing 75.

### DETAILED EMBODIMENTS

In order to make the purpose, the technical solution and the benificial effect of the present invention more clearly under understood, the specific embodiments of the present invention are further illustrated in detail below in conjuction with accompanied drawings. The illustrative embodiments and the description thereof of the present invention herein are used for explanation of the present invention, without limiting the present invention.

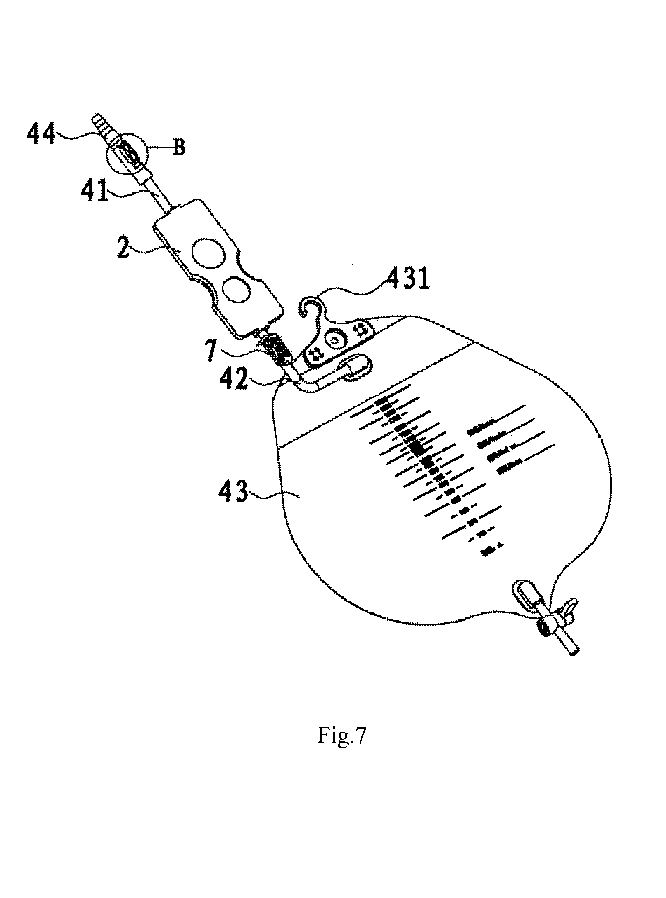

As is shown in figure 7, a urine drainage device comprises a urine inlet 31 capable of connecting to the urinary catheter which is inserted into the human body, and a urine outlet 32, the urine outlet 32 can connect to the urine storage bag 43 which stores urine, and can also connect to other components that can hold urine, i.e. the urine drainage device has the function capable of outputing the urine in the bladder of human body.

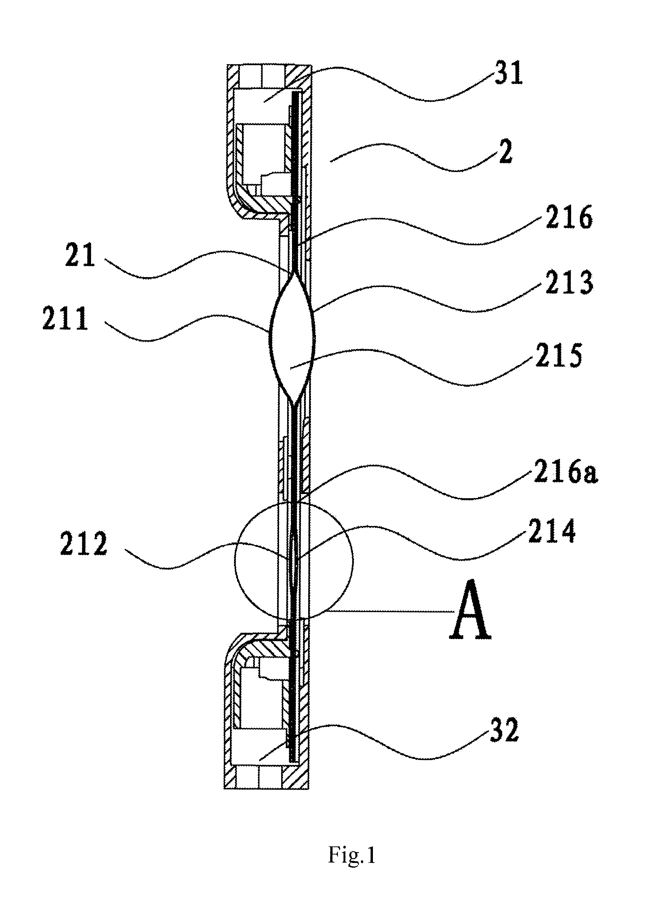

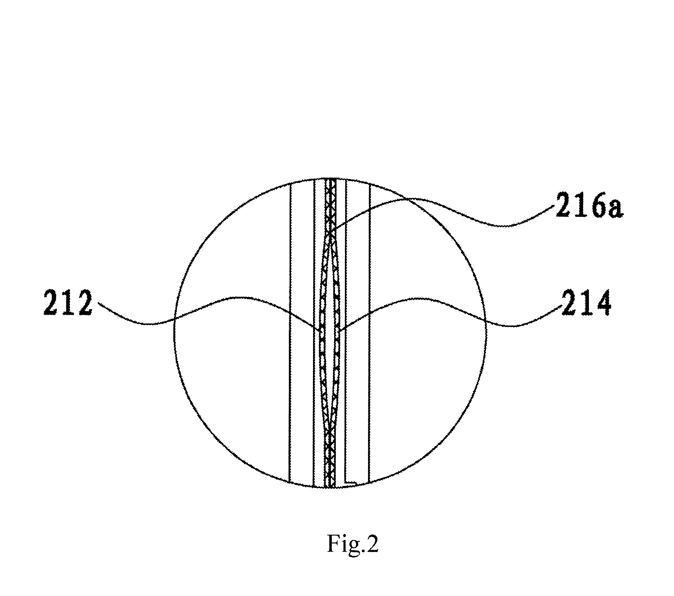

As is shown in figure 1, the urine drainage device further comprises a bionic bladder unit 2, the bionic bladder unit 2 comprises a main body component 21 capable of forming the urine storage cavity 215 which stories urine, the urine storage cavity 215 is connected to the urine inlet 31 and the urine outlet 32 via a connecting tube 216, and when the communication between the urine storage cavity 215 and the urine outlet 32 is blocked, the urine inlet 31 and the urine outlet 32 are shut off, so that the urine cannot discarge. There are various manners for blocking, e.g. setting a manually controlled valve 7, etc. The main body component 21 comprises a first pressure transmission surface 211 and a second pressure transmission surface 213 capable of deforming along with the change of the urine pressure in the urine storage cavity 215, the first pressure transmission surface 211 and the second pressure transmission surface 213 can be made of elastic material polyvinyl chloride by injection molding, made as a whole with the main body component 21, and also can be made by hermetically sealed bonding with the main body component 21 using medical adhesive. As is shown in figure 2, the lower connecting tube 216a which connects the urine storage cavity 215 and the urine outlet 32 is provided with a first action area 212 and a second action area 214 which can be blocked by the action of an external force, the first action area 212 and the second action area 214 can be made of flexible material polyvinyl chloride by injection molding, made as a whole with the main body component 21, and also can be made by hermetically sealed bonding with the main body component 21 using medical adhesive. The first action area 212 and the second action area 214 are oppositely disposed; and when the first action area 212 and the second action area 214 are applied by external forces simultaneously, the communication between the urine storage cavity 215 and the urine outlet 32 is blocked; according to the deformation of the first action area 212 and/or the second action area 214, the external force applied on the first action area 212 and/or the second action area 214 can be removed, and when either external force applied on the first action area 212 and the second action area214 is removed, the blockage between the urine storage cavity 215 and the urine outlet 32 can be removed, so that the communication between the urine storage cavity 215 and the urine outlet 32 can be restored. Because the urine drainage device is provided with a first pressure transmission surface 211 and a second pressure transmission surface 213, it can be determined whether to communicate the micturition according to the deformation of the first pressure transmission surface 211 and/or the second pressure transmission surface 213 regardless of employing manually controlled, electrically controlled, or mechanically controlled manner, thereby achieving the function of helping the patient to micturate. When manually controlled manner is employed, whether the patient needs to micturate can be judged according to the deformation level of the pressure transmission surface, thereby increasing the flexibility. Meanwhile, the urine drainage device in this structure has the advantages of having a simple structure and being suitable for industrial production, etc.

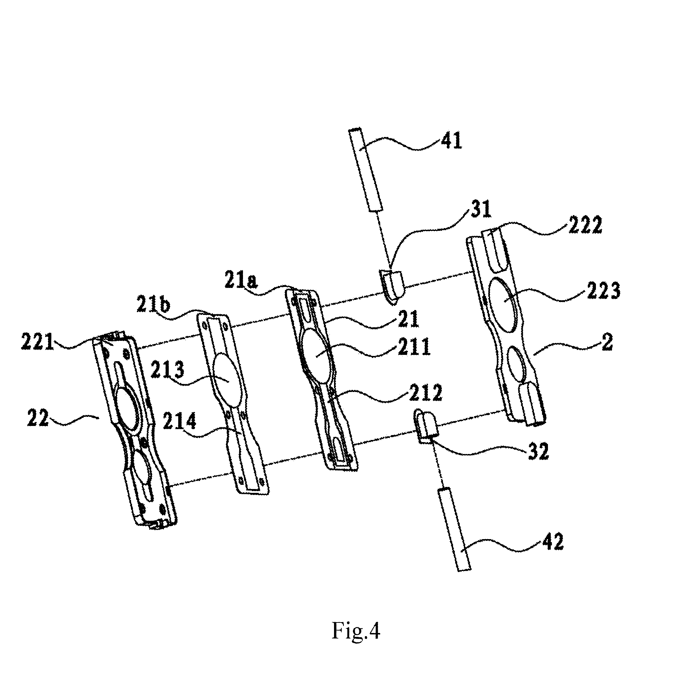

Specifically, the main body component 21 comprises two diaphragms, i.e. the first diaphragm 21 a and the second diaphragm 21b as shown in figure 4. The first diaphragm 21 a is formed with a first pressure transmission surface 211 and a first action area, the second diaphragm 21b is formed with a second pressure transmission surface 213 and a second action area 214, the first action area 212 and the second action area are oppositely disposed and form a lower connecting tube 216a when they are fastened and sealed; the first pressure transmission surface 211 and the second pressure transmission surface 213 are oppositely disposed, forming the above-mentioned urine storage cavity 215. The sealing of the two diaphragms can be achieved by manners such as ultrasonic welding or heat sealing process.

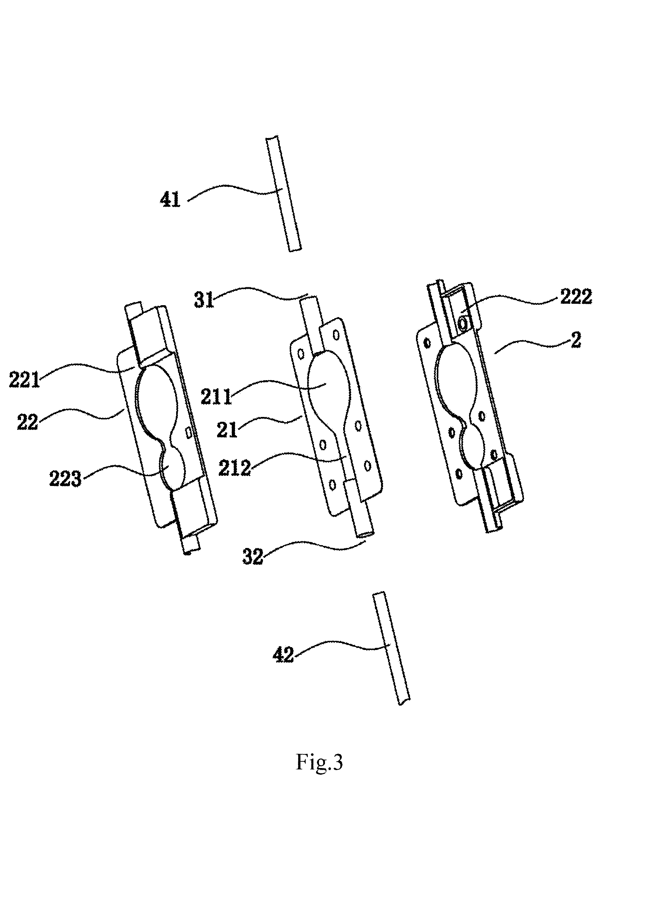

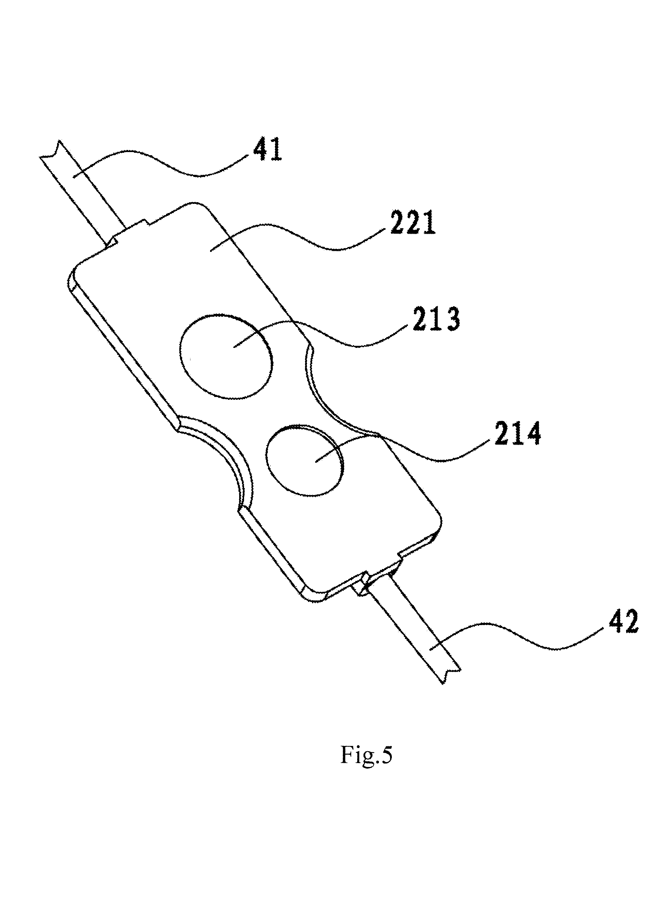

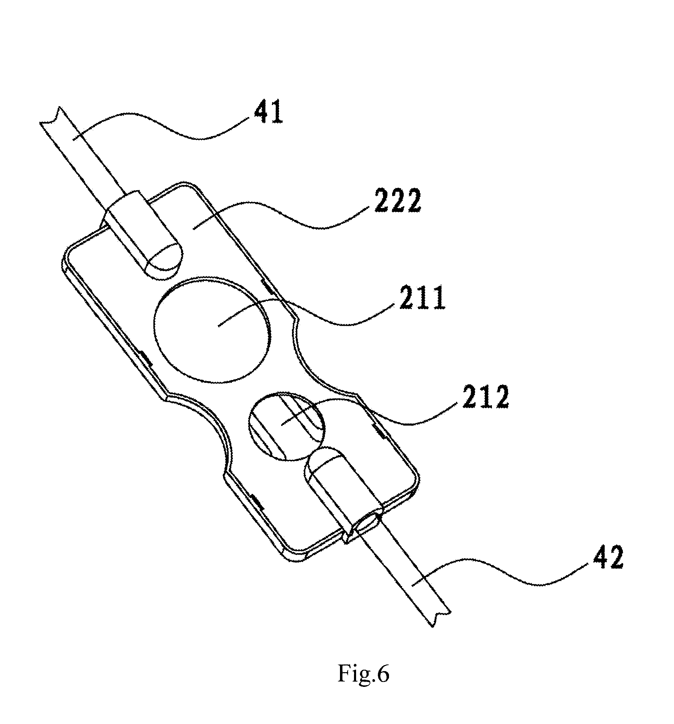

In order to protect the diaphragms from being damaged by external force, the bionic bladder unit 2 further comprises a cover housing 22 made of hard material, as is shown in figures 3 and 4. The cover housing is divided into a right cover housing 222 and a left cover housing 221. The two components match and house on the main body component 21, and sandwich the main body component 21, and after the two cover housings are fastened, they are capable of housing the portions on the main body component 21 except the first pressure transmission surface 211, the first action area 212, the second pressure transmission surface 213 and the second action area 214. The cover housing 22 is provided with a bare hole 223 which bares the first pressure transmission surface 211, the first action area 212, the second pressure transmission surface 213 and the second action area 214, so as to avoid interfering the deformation of the pressure transmission surface and the blockage to the action area. As is shown in figures 5 and 6, the cover housing 22 can play better supporting, position-fixing and protective effects on the main body component 21, so that the urine drainage device has certain capability against damage by external force. The cover housing 22 can specifically be made of the material of acrylonitrile-butadiene-styrene by injection molding.

Specifically, the lower connecting tube 216a connecting the urine storage cavity 215 and the urine outle 32 is in an elongated tubular shape, the urine storage cavity 215 is in an oval shape, the first pressure transmission surface 211 and the second pressure transmission surface 213 are also in an oval shape. The shape of the above-mentioned pressure transmission surface is designed to more significantly highlight the deformation of the pressure transmission surface, and the elongated design of the lower connecting tube 216a is convinient for its blockage by external force.

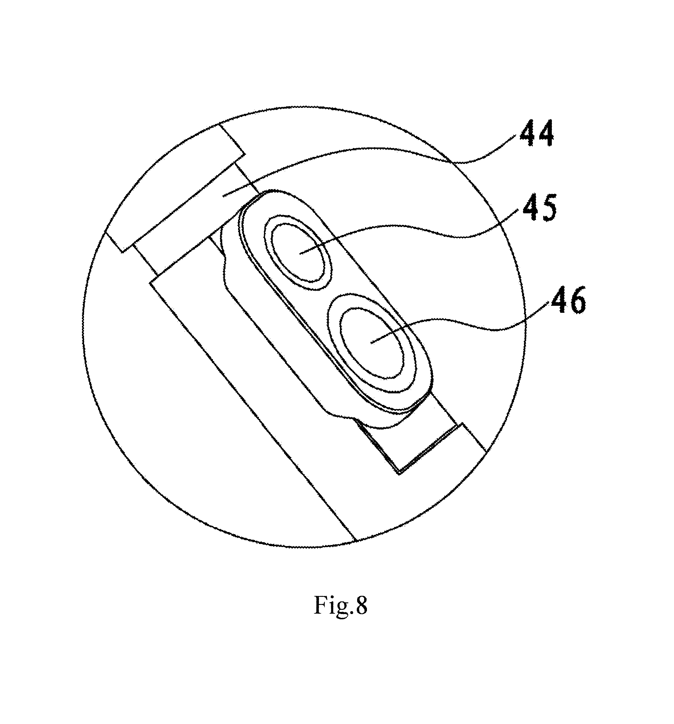

With respect to each exapmple mentioned above, to facilitate the communication of the bionic bladder unit 2 and the urine storage bag 43 as well as the urinary catheter, the urine inlet 31 and urine outlet 32 can connect to the urinary inlet tube 41 and urinary outlet tube 42 respectively, wherein the urinary inlet tube 41 is communicated with the urinary catheter, and the urinary outlet tube 42 is communicated with the urine storage bag 43. Both the urinary inlet tube 41 and the urinary outlet tube 42 can be provided with a manually controlled valve 7, so that they can be blocked by clamping the tube if required. As is shown in figure 8, the urinary inlet tube 41 is provided with a urinary catheter connector 44 for connecting to the urinary catheter, the urinary catheter connector 44 or the urinary inlet tube 41 is provided with a self-sealable region for temperature collecting 45 and/or urine sampling 46, the self-sealable region for temperature collecting 45 can be penetrated by a sharp temperature collecting neddle, so as to contact the urine and perform temperature colleting at the temperature measuring point. Furthermore, when the temperature collecting needle is extracted after completing the collecting, due to the flexible self-sealable region, the inserted position will automatically recover, i.e. it has the function of automatically sealing, thereby maintaining a good sealing nature, and preventing the leakage of urine. The self-sealable region for urine sampling 46 also has the technical effects as mentioned above.

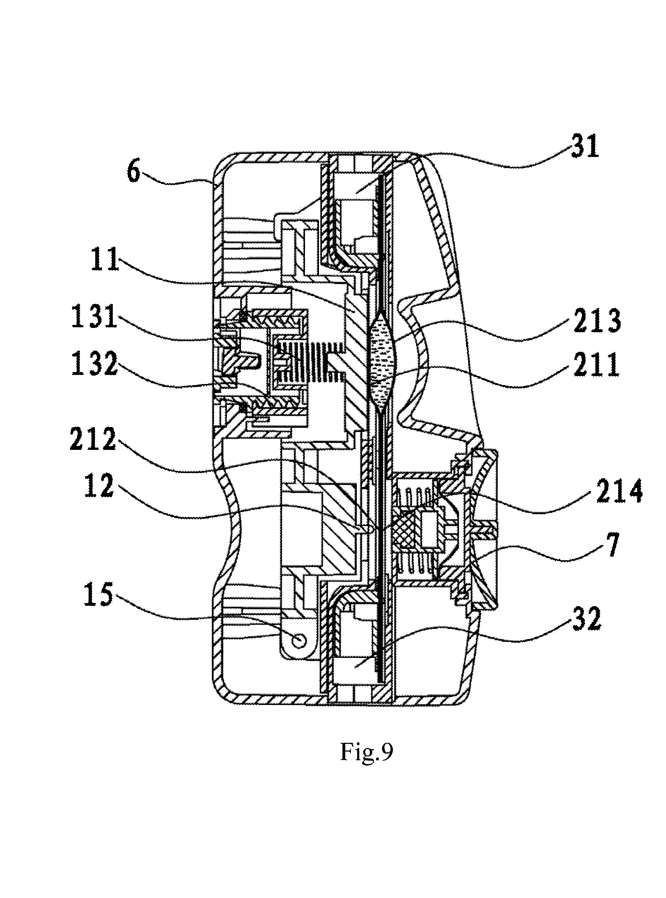

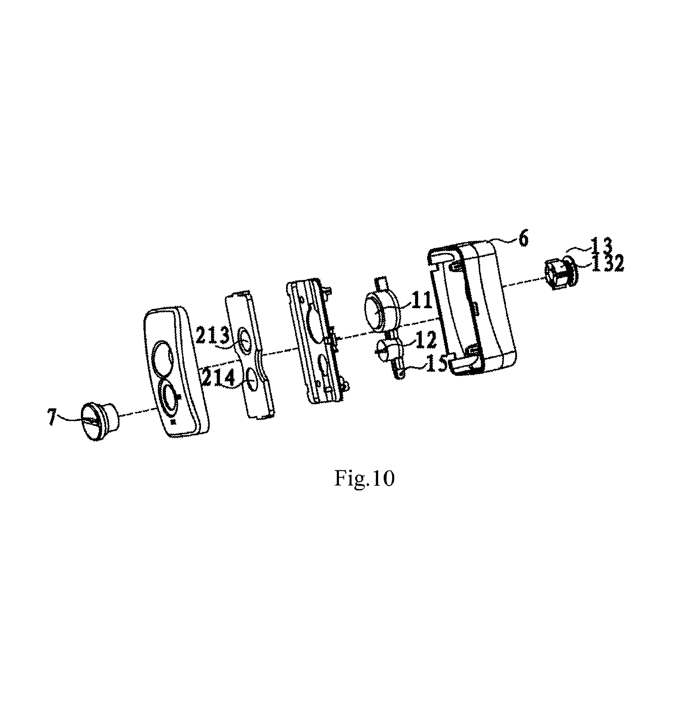

As is shown in figures 9-13, the present invention further provides a unrine drainage control device, comprising the abov-mentioned urine drainage device, and further comprising a control portion. The control portion comprises a pressure transmission component 11 and a closure component 12, both of them can be a unitary setting or a split setting. The pressure transmission component 11 is capable of contacting with the deformed first pressure transmission surface 211 and/or second pressure transmission surface 213, and shifts in position by the action of the force produced by the deformation of the first pressure transmission surface 211 and/or the second pressure transmission surface 213; the closure component 12 is capable of shifting along with the shift in position of the pressure transmission component 11, and when shifting in position, the closure component 12 is capable of applying or removing the exteranal force acted on the first action area 212 and/or the second action area 214, thereby blocking or unblocking the communication between the urine storage cavity 215 and the urine outlet 32.

In one status: when the device is initially used, the closure component 12 blocks the communication bewteen the urine storage cavity 215 and the urine outlet 32, and when postural changes, coughing, bladder spasms and other factors occur in a patient, the pressure in the bladder transiently increases and exceeds the safe value that can be afforded by the bladder, the force produced by the deformation of the pressure transmission surface is capable of driving the pressure transmission component 11 to shift, the pressure transmission component 11 further drives the closure component 12 to shift, the closure component 12 removes the external force applied on the the action area, the urine storage cavity 215 and the urine outlet 32 communicate, and the urine are micturated to release pressure, thereby protecting the bladder from damage, and preventing the occurrence of dangerous conditions such as urine reflux and overflow of urine; when the pressure in the bladder reduces below the safe value of the bladder, the deformation level of the pressure transmission surface reduces, the pressure transmission component 11 and the closure component 12 also recover correspondingly, the closure component 12 continues to apply external force on the action area, so as to block the communication between the urine storage cavity 215 and the urine outlet 32. The pressure transmission surface mentioned above can be selected to be the first pressure transmission surface 211 or the second pressure transmission surface 213.

In another status: when the device is initially used , the closure component 12 applies external forces to the action area so as to block the communication between the urine storage cavity 215 and the urine outlet 32, and with the increasing of urine in the urine storage cavity 215, the first pressure transmission surface 211 and/or the second pressure transmission surface 213 gradually deform, and whether to operate the manually controlled valve 7 to open the comunication between the urine storage cavity 215 and the urine outlet 32 is determined according to the deformation level of the pressure transmission surface observed by the naked eyes of human. This type of setting makes it possible to micturate when the urine pressure in the bladder achieves a certain value, thereby training the the effects of the bladder to perceive the filling of urine in the bladder and to achieve the urinary reflex, so as to ahieve tha effect of managing the micturition activity of the bladder, and finally achieving the vuluntary micturition of the bladder.

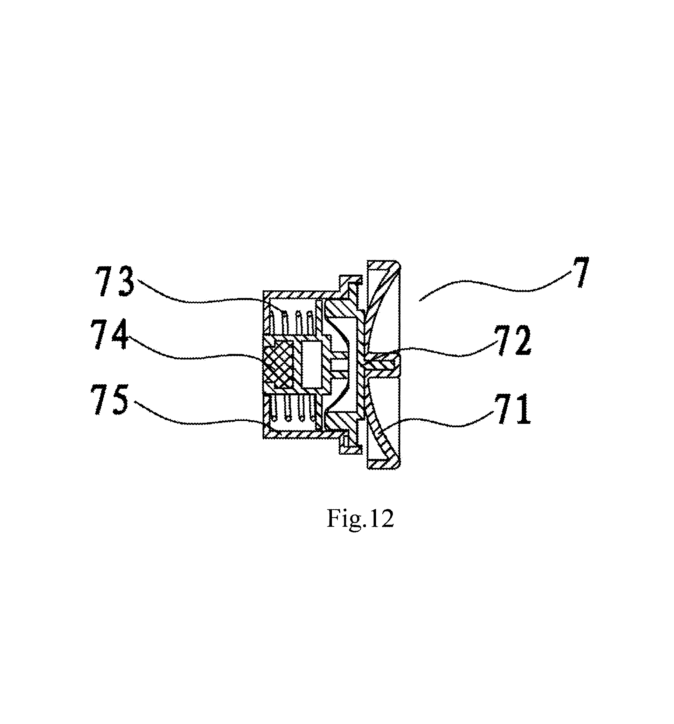

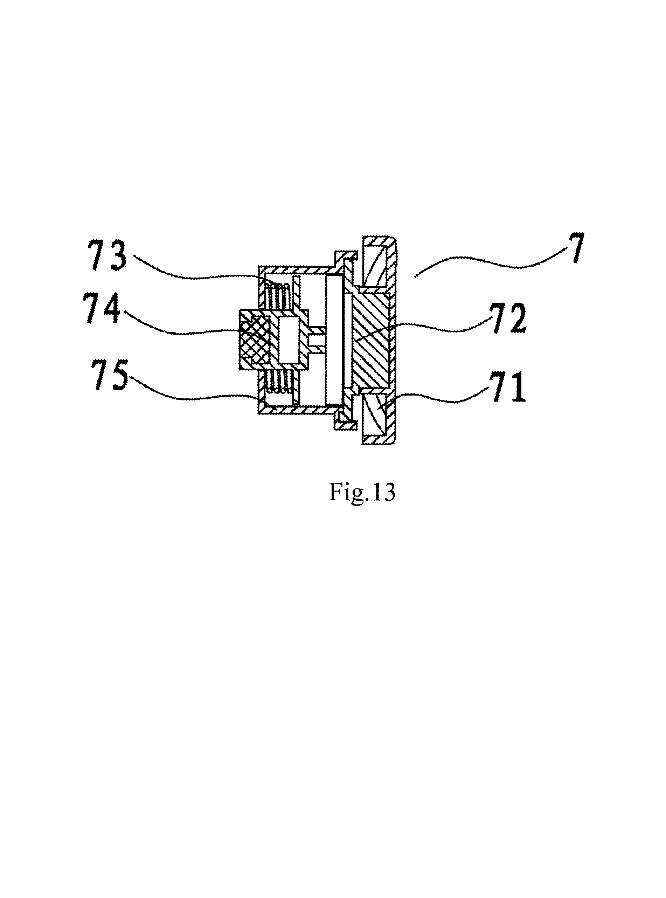

As is shown in figures 12 and 13, the manually controlled valve 7 is located on the urine drainage control device, comprises a rotation handle 71, a valve main body 72, a reset component 73, a valve spool 74 and a valve housing 75, and the working principle thereof is as follows: utilizing the rotation handle 71 to drive the valve main body 72, which can push or lift pushing the valve spool 74, and then utilizing the valve spool 74 to block or unblock the the first action area 212 or the second action area 214, and when blocking the first action area 212 or the second action area 214, the handle 71 rotates the valve main body 72 to make the valve spool 74 protrude out of the valve housing 75, as is shown in figure 13; when unblocking the first action area 212 or the second action area 214, the handle 71 rotates the valve main body 72 to make the valve spool 74 retreat inside the valve housing 75, and the reset component 73 can help the valve spool 74 to retreat inside the valve housing 75, as is shown in figure 12.

The urine drainage control device employs a mechanical principle to control the releasing of pressure and the micturition, protects the bladder form damage, to achieve the technical effects of preventing reflux and preventing overflow of urine, and achieves the function of simulating the normal physiological micturition in human body according to the pressure in the bladder, and the operation is simple, and easy for the patients themselves and medical staff to operate.

There is a gap between the pressure transmission component 11 and the pressure transmission surface, and when the pressure transmission surface deforms along with the increasing pressure in the urine storage cavity 215, the gap can be tapered until eliminated, i.e. when the urine pressure in the urine storage cavity 215 reaches a certain level, the deformation level of the first pressure transmission surface 211 or the second pressure transmission surface 213 can satisfy to achieve contacting with the pressure transmission component 11, thereby driving the pressure transmission component 11 to shift.

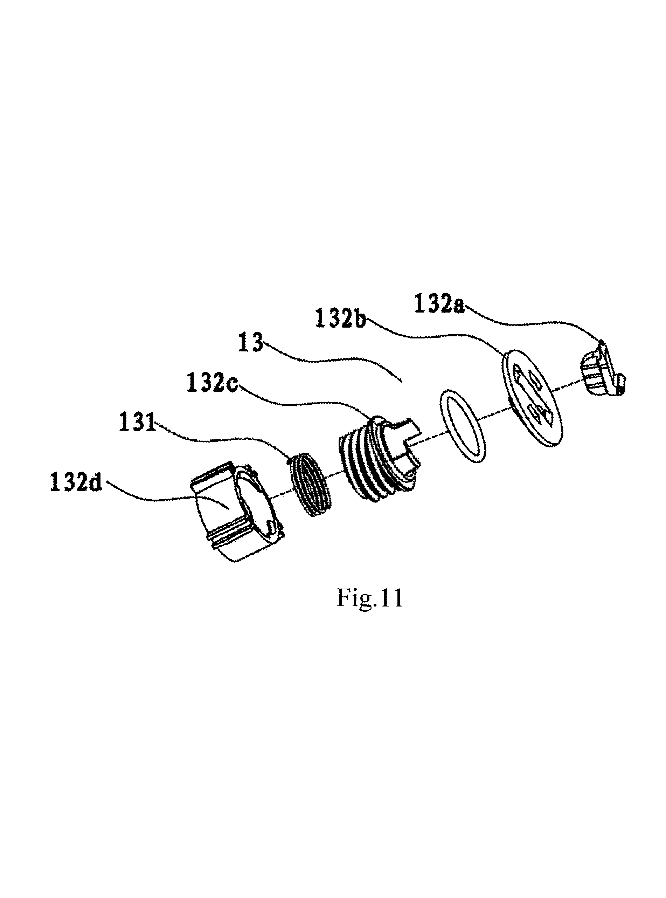

The control portion of the urine drainage control device further comprises a resistance component 13, as is shown in figure 11, the resistance component 13 applies a predetermined resistance to the pressure transmission component 11 and/or the closure component 12 to prevent the deformation of corresponding pressure transmission surface, the predetermined resistance can be set to be 40 cmH₂O which is a safe pressure value for the bladder in human body, and can also be set individually according to the conditions of the bladder function of the patient. When the force of the corresponding pressure transmission surface acted on the pressure transmission component 11 or the closure component 12 is greater than the predetermined resistance, the pressure transmission component 11 drives the closure component 12 to shift in position, removes the blockage to the communication between the urine storage cavity 215 and the urine outlet 32, thereby performing the pressure releasing and the micturition. The resistance of the resistance component 13 is produced by the spring 131, one end of the spring 131 can connect to housing 6, and the other end connects to the pressure transmission component 11 or the closure component 12, the spring 131 can be in a pre-compressed state, and only when overcoming the force of the spring, the acting forces of the deformation of the pressure transmission surface on the pressure transmission component 11 can drive the shift of the pressure transmission component 11, thereby achieving the starting of the urine drainage device to release pressure and to micturate; the resistance component 13 further comprises an adjustment portion 132, the adjustment portion 132 comprises a handle 132a, an index plate 132b, a right threaded housing 132c and a left threaded housing 132d, the handle 132a is utilized to drive the right threaded housing 132c to rotate inside the left threaded housing 132d, to compress or release the spring 131 and to adjust the length thereof, thereby adjusting the predetermined resistance applied to the pressure transmission component 11 and/or the closure component 12, and the index plate 132b can display the adjustment value of the predetermined resistance.

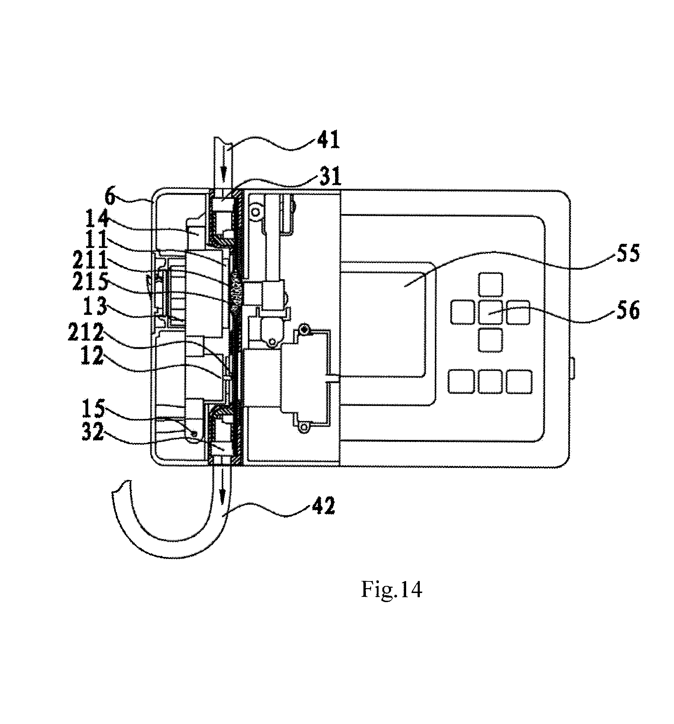

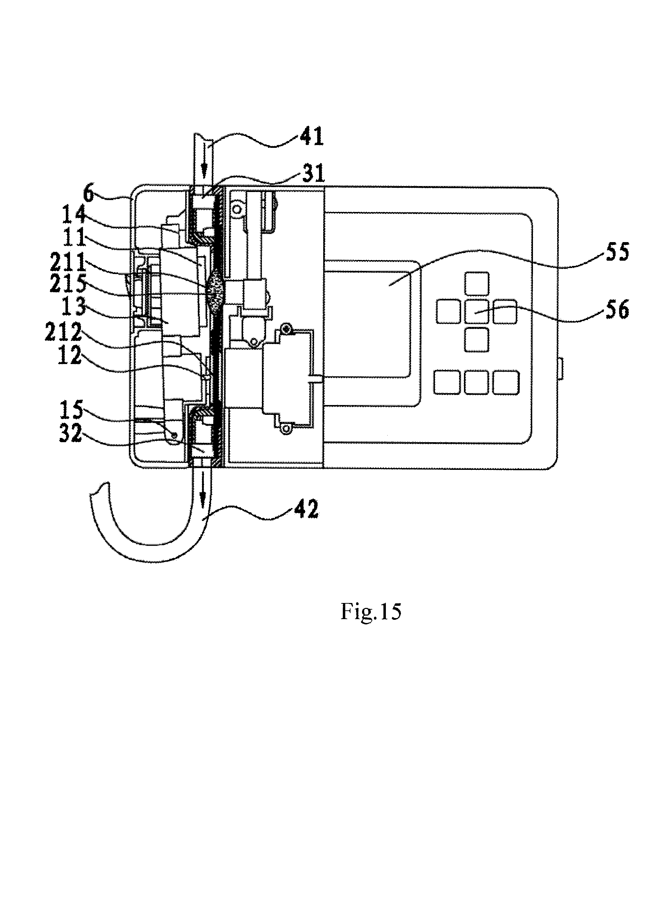

As is shown in figure 14, the pressure transmission component 11 and the closure component 12 are a unitary structure, and are made as a whole by injection molding; the control portion further comprises a rotation axis 15, and when the pressure transmission component 11 shifts in position along with the deformation of the first pressure transmission surface 211, the pressure transmission component 11 and the closure component 12 are capable of rotating together around the rotation axis 15. In figure 14, because the accumulated urine in the bladder is little and does not reach the predetermined resistance value for the resistance component 13, the pressure transmission component 11 does not shift in position, the closure component 12 continues to apply an external force acted on the first action area 212, so that the urine cannot flow out from the urine storage cavity 215 via the urine outlet 32. As is shown in figure 15, due to the sudden rise in pressure in the bladder, the first pressure transmission surface 211 overcomes the resistance of the resistance component 13, drives the pressure transmission component 11 to rotate counterclockwise, the closure component 12 also rotates counterclockwise correspondingly, and the closure component 12 no longer blocks the communication between the urine storage cavity 215 and the urine outlet 32; when the pressure in the bladder reduces to a certain value, the spring 131 restores and drives the pressure transmission component 11 to rotate clockwise, the closure component 12 rotates clockwise correspondingly, thereby blocking the communication between the urine storage cavity 215 and the urine outlet 32.

The pressure transmission component 11 and the closure component 12 herein can also be a split structure, as long as both of them remain relatively fixed. In addition, the pressure transmission component 11 can also be provided with a limit component 14, which can be a portion on the pressure transmission component 11, for limiting the pressure transmission component 11 to shift within a certain rainge or to locate at a certain position after the shifting of the pressure transmission component 11, so that the closure component 12 remove the closure to the first action area 212 within a period of time.

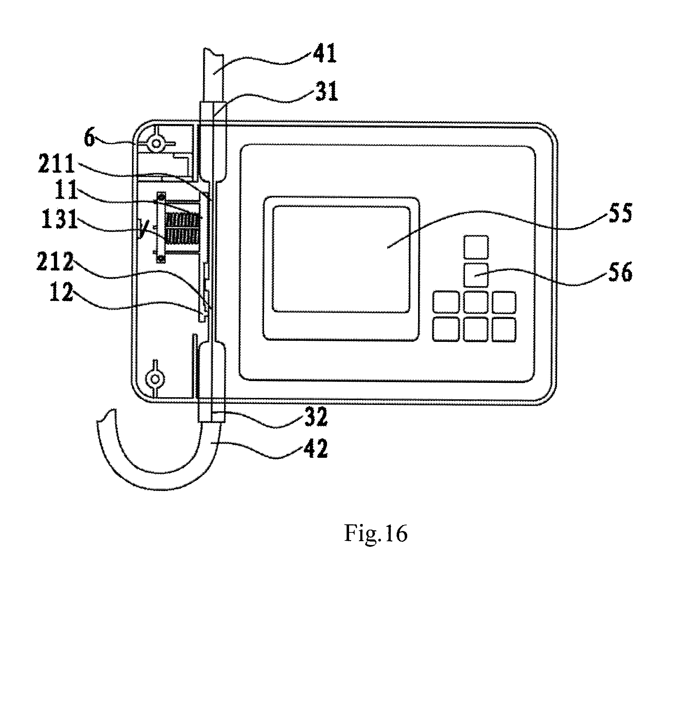

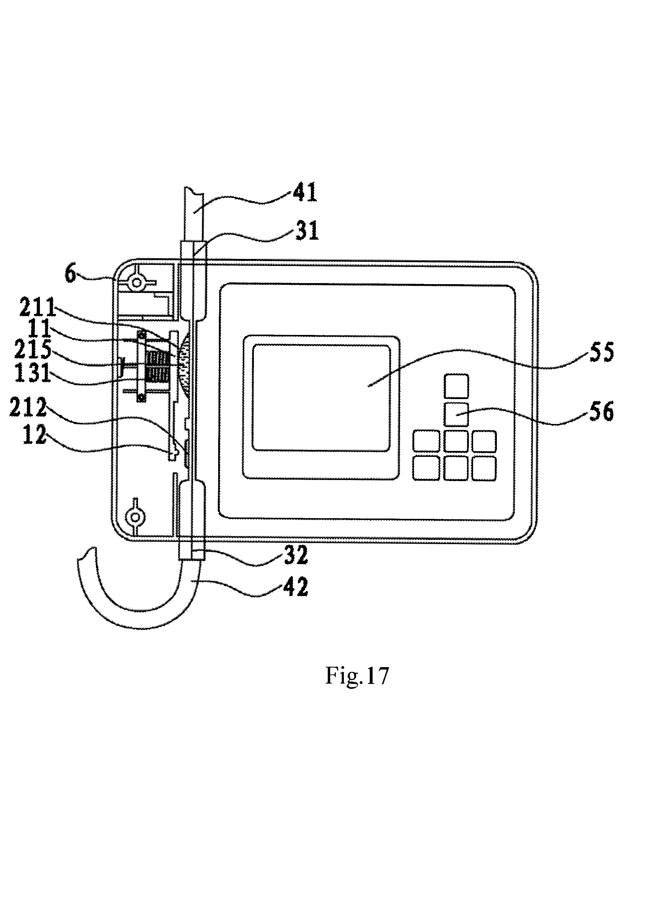

As is shown in figures 16 and 17, the pressure transmission component 11 and the closure component 12 are a unitary structure; the pressure transmission component 11 is provided with a guide rod, the housing 6 is provided with a socket, the guide rod is capable of shifting in the socket in the stretching direction of the spring 131. The guide rod and the socket are mainly used to define the shifting direction of the pressure transmission component 11 and the closure component 12, so that both of them can only shift in the stretching direction of the spring 131. As is shown in figure 16, both ends of the pressure transmission component 11 are provided with a guide rod respectively, as a matter of fact, it may be one or more guide rods; the spring 131 is set parallel to the guide rod, so that the guide rod can only shift in the stretching direction of the spring 131. The guide rod is substantially perpendicular to the pressure transmission component 11, when the spring 131 stretches out, the pressure transmission component 11 is compacted onto the first pressure transmission surface 211, by the time, the closure component 12 is compacted to the first action area 212, and applies blocking to the communication between the urin storage cavity 215 and the urine outlet 32; after the first pressure transmission surface 211 pushes the pressure transmission component 11 to shift, the closure component 12 gradually leaves the first action area 212, thereby removing the blockage and achieving the pressure releasing and the micturition. As is shown figure 17, after the releasing pressure and the micturition, the spring 131 restores, and recovers to a blocked state.

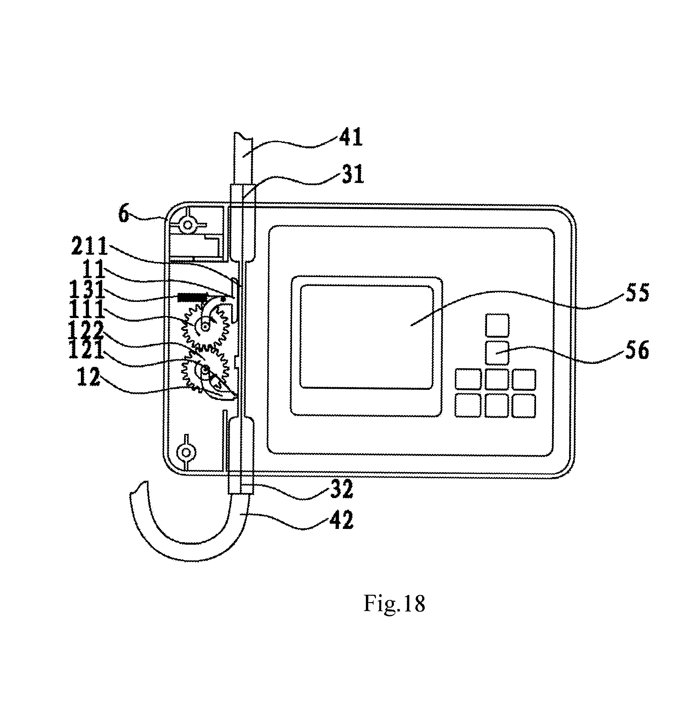

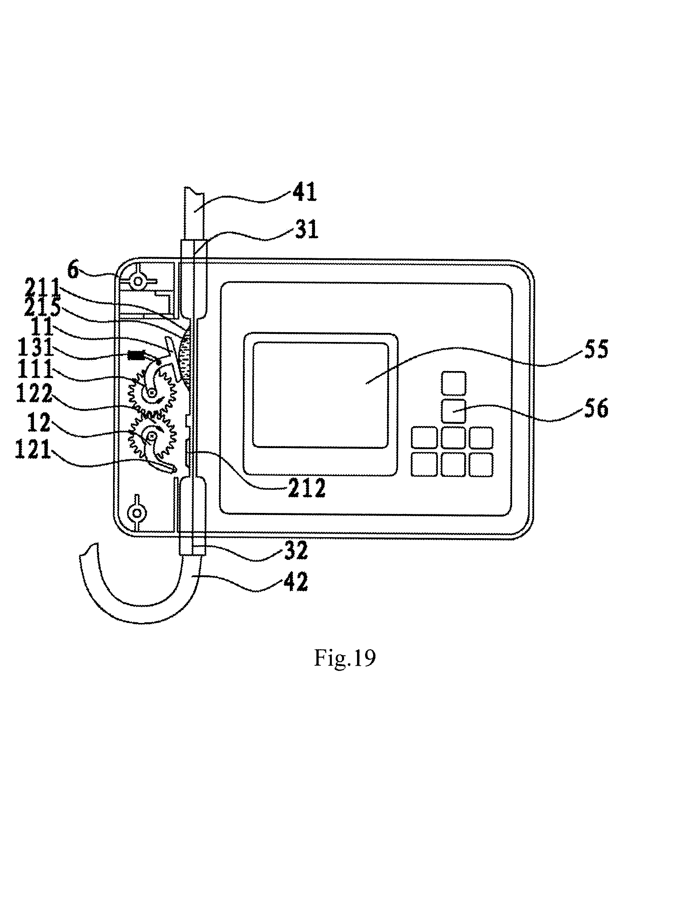

As is shown in figure 18, the pressure transmission component 11 comprises a first gear 111, the closure component 12 comprises a closure head 121 and a second gear 122 engaged to the first gear 111, the first gear 111 and the second gear 122 are all fixed with respect to the housing 6; the spring 131 and the pressure transmission component 11 are connected through a movable axis, the pressure transmission component 11 comprises a contact plate that can contat with the pressure transmission component 211, the contact plate is fixed to the first gear 111, and when the contact plate shifts, it drives the first gear 111 to rotate, the first gear 111 drives the second gear 122 to rotate, and when the second gear 122 rotates, it is capable of driving the closure head 121 to shift, thereby unblocking the communication between the urine storage cavity 215 and the urine outlet 32, and achieving the pressure releasing and the micturition. As is shown in figure 19, after the pressure releasing and the micturition, the spring 131 restores, and recovers to a blocked state.

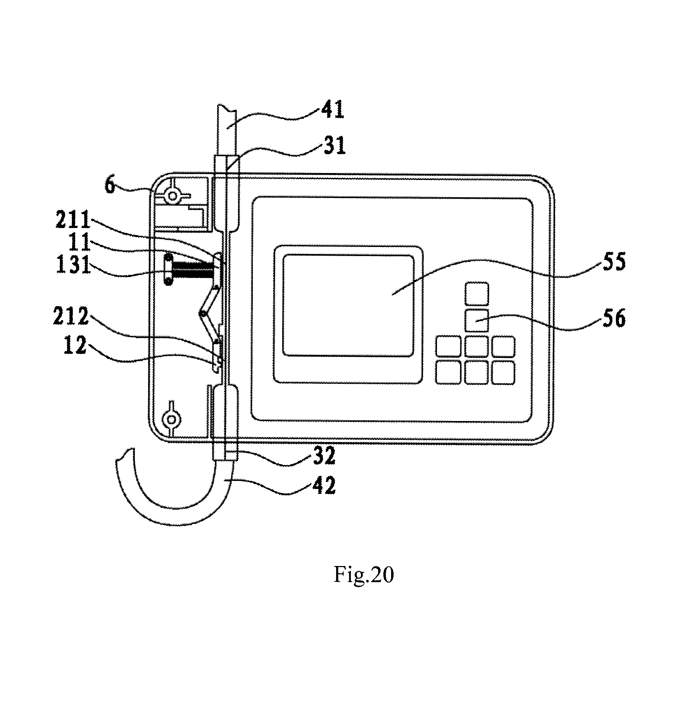

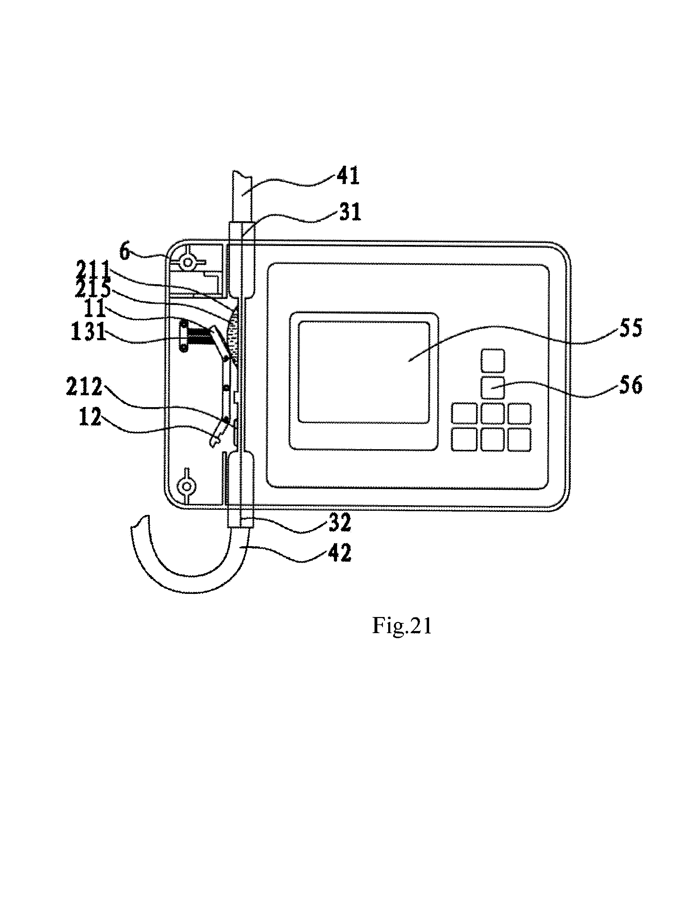

As is shown in figure 20, the pressure transmission component 11 and the closure component 12 are hinged, and when the pressure transmission component 11 shifts in position along with the deformation of the first pressure transmission surface 211, it drives the closure component 12 to rotate so as to block or unblock the communication between the urine storage cavity 215 and the urine outlet 32, i.e. the pressure transmission component 11 and the closure component 12 form a link mechanism, thereby achieving linkage. The pressure transmission component 11 is bent like a lever, comprises a linkage segment and a contact segment for contacting the first pressure transmission surface 211; the closure component 12 is also bent like a lever, comprises a likage segment and a closure segment for applying external force to the first action area 212. When blocked, the linkage segment of the pressure transmission component 11 and the closure component 12 are connected and tilt; when the contact segment of the pressure transmission component 11 shifts and tilts, the closure segment of the closure component 12 also tilts, thereby unblocking the communication between the urine storage cavity 215 and the urine outlet 32. As is shown in figure 21, after releasing pressure and micturating, the spring 131 restores, and recovers the blocked state.

The housing 6 on the urine drainage control device can be provided with a positioning projection, the cover housing 22 of the urine drainage device is provided with a positioning slot fitted to the positioning projection. This type of setting manner simply makes the installation of the urine drainage device be more convenient, rapid, and easy to dissambly.

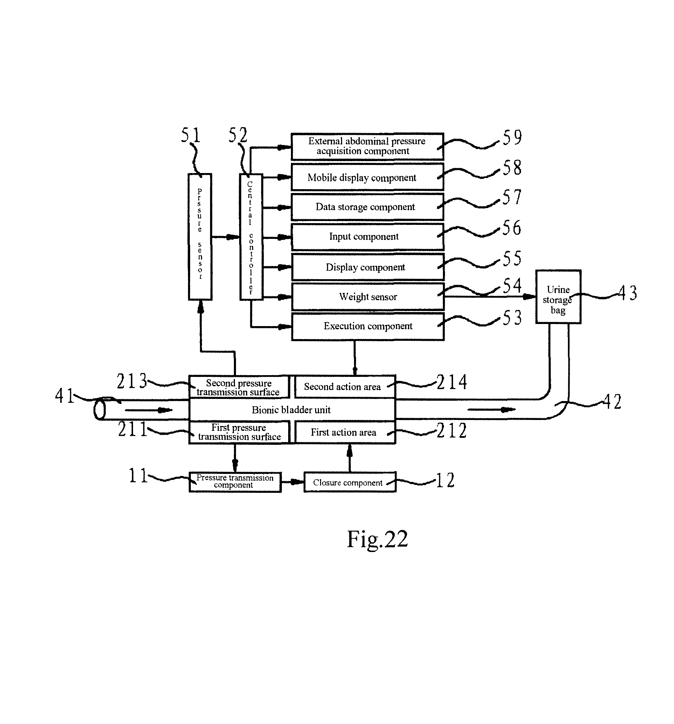

The present invention further provides a urine drainage control system, as is shown in figure 22: comprising a urine drainage control portion and an electric control portion, the urine drainage control portion is the urine drainage control device in the example mentioned above, the pressure transmission component 11 and the closure component 12 respectively act on the first pressure transmission surface 211 and the first action area 212. The electric control portion comprises a central controller 52, an execution component 53, and a pressure sensor 51 that monitors the bladder pressure, the central controller 52 receives the pressure signal outputted by the pressure sensor 51, when the bladder pressure is equal to or greater than the micturition threshold and the duration is equal to or greater than the judgement time, the central controller 52 outputs an instruction of communicating the urine storage cavity 215 and the urine outlet 32 to the execution component 53; the pressure sensor 51 and the execution component 53 respectively act on the second pressure transmission surface 213 and the second action area 214. The urine drainage control system further comprises a weight sensor 54, a display component 55, an input component 56, a data storage component 57, a mobile display component 58 and an external abdominal pressure acquisition component 59. The weight sensor 54 is used for real-time monitoring the weight of urine in the urine storage bag 43 and transporting the weight signal to the central controller 52. The weight sensor 54 is connected to the hook 431 of the urine storage bag 43, and when the urine weight exceeds the preset value, the central controller 52 can send out a warning signal to change the urine storage bag 43, and the central controller 52 is capable of plotting a series of curves reflecting the bladder functions, such as urine flow rate curve, urinary output and weight change curve, according to the parameters such as weight signal, micturition time and urine density. The display component 55 is connected to the central controller 52, for displaying the monitored data and the artificially set data as well as the calculated result of the central controller 52; the input component 56 is connected to the central controller 52, for setting parameter and/or querying the stored record of the data storage component 57 and the calculated result of the central controller 52; the data stroage component 57 is used for the output of data and the input of data, the data storage component 57 is capable of storing the parameter set by the input component 56, as well as the urine flow rate curve, the urinary output curve, the bladder pressure curve and the abdominal pressure curve that are anlyzed and generated by the central controller 52, and is capable of transporting the above-mentioned curves to an external device, and is capable of further tranporting the parameter recorded on the external device to the central controller; the external abdominal pressure acquisition component 59 can collect the abdominal pressure in the human body, and transports the collected signal to the central controller 52, the central controller 52 is provided with a connector connected to the external abdominal pressure acquisition component 59; the mobile display component 58 is wirelessly connected to the central controller 52, for displaying the contents displayed by the external abdominal pressure acquisition component 59. This system can be powered by a battery or powered by a fixed power source.

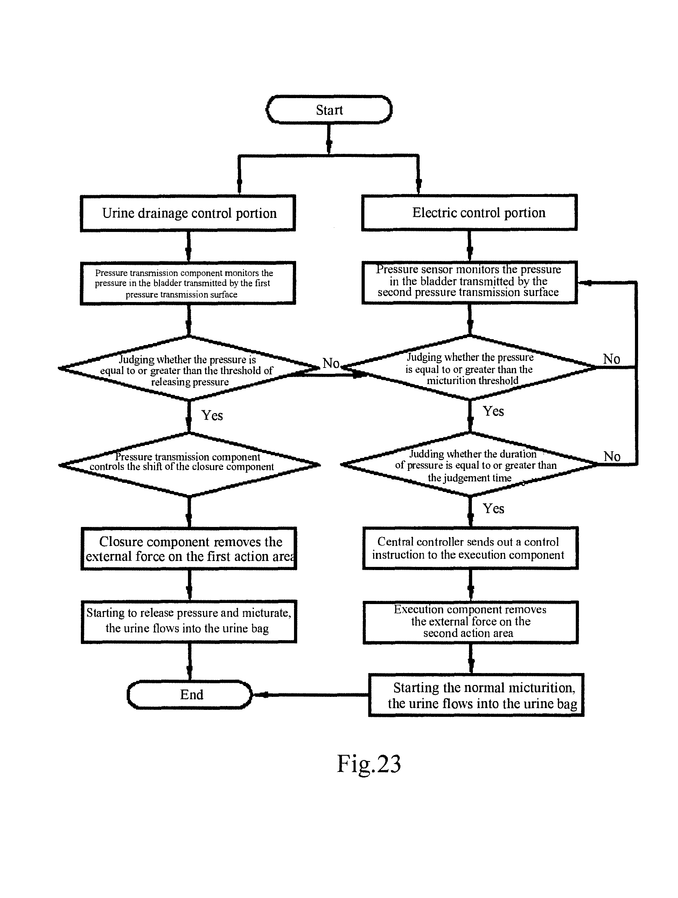

The working flow chart of the above-mensioned urine drainage control system is as shown in figure 23, and when the bladder pressure transmitted by the second pressure transmission surface 213 monitored by the pressure sensor 51 is equal to or greater than the micturition threshold, and the duration is equal to or greater than the judgement time, the central controller 52 sends instruction to the execution component 53, the execution component 53 removes the external force acted on the second action area 214, the urine storage cavity 215 communicates with the urine outlet 32, simulates the normal physiological micturition of human body, and the time for micturition is the preset micturition duration. The micturition threshold can be set to the normal human bladder micturition threshold, about 20 cmH₂O, and can also set the threshold according to the bladder functional status of the patient; the judgement time is a time set according to the bladder functional status of the patient, and is generally set for about 10 sec; the preset micturition duration is also set according to the bladder functional status of the patient, and is generally set for any value between 10 sec and 300 sec, or set individually according to the need of the patient. After completing the process of micturition, the execution component 53 continues to apply the external force acted on the second action area 214, and blocks the communication between the urine storage cavity 215 and the urine outlet 32. When the pressure in the bladder of the patient transiently increases, the first pressure transmission surface 211 of the urine drainage control portion of the urine drainage control system deforms, so that the pressure transmission component 11 drives the closure component 12 to remove the external force applied on the first action area 212, and communicates the the urine storage cavity 215 with the urine outlet 32, the urine flows through the urine outlet 32 and enters the urine storage bag 43, i.e. serving an effect of instantly releasing pressure, and the the communication between the urine storage cavity 215 and the urine outlet 32 is blocked again after the pressure reduces. The urine drainage control system equals to a combination of electrical control and mechanical control, which, in one aspect, is capable of simulating the normal micturition of the physiological function of human body by utilizing the electric control portion, and in another aspect, prevents the risks of urnine reflux and overflow of urine caused by pseudo transiently increased bladder pressure by utilizing the mechanical portion.

The above-mentioned execution component 53 comprises an air pump and an air sac, the air sac is capable of applying an external force to the second action area 214, blocks the communication between the urine storage cavity 215 and the urine outlet 32, and can also execute the control signal of releasing air sent by the central controller 52, to remove the external force applied on the second action area 214, and comunicates the urine storage cavity 215 with the urine outlet 32, to perform normal micturition. After the completing of the micturition process, the air pump inflates the air sac, to restore blocking the communication between the urine storage cavity 215 and the urine outlet 32.

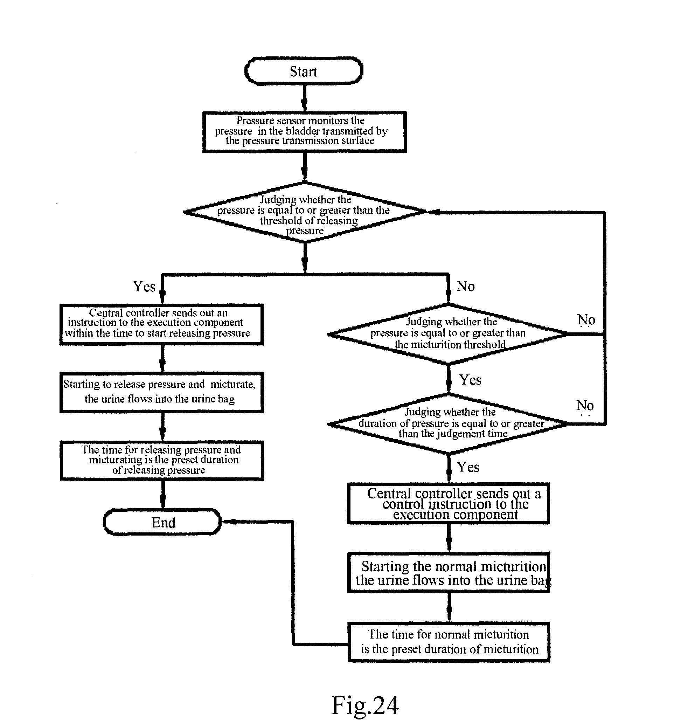

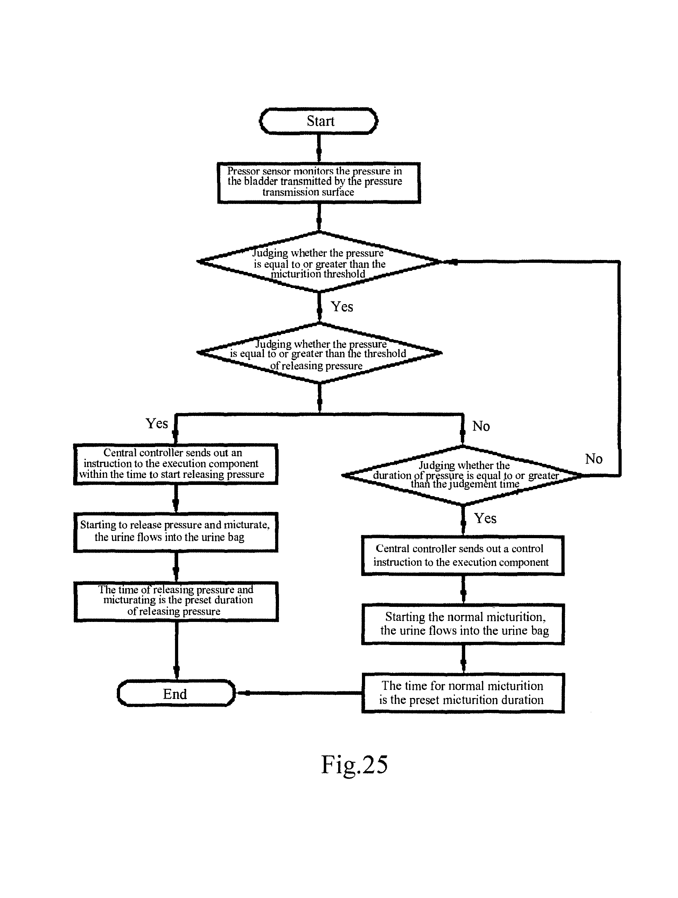

A micturition method that protects the bladder function, as is shown in figures 24 and 25: presetting a threshold of releasing pressure, a micturition threshold and a duration of releasing pressure, a micturition duration, a judgement time, a time to start releasing pressure, and the threshold of releasing pressure can be set to the safe pressure value of 40 cmH₂O, or set to any value between the safe pressure value and the micturition threshold. In addition, because the setting of the threshold of releasing pressure can effectively protect the bladder from damage, the threshold of releasing pressure can be set to a value greater than the safe pressure value 40 cmH₂O for the bladder according to the functional status of individual bladder; the duration of releasing pressure is a time set according to the functional status of a patient's bladder, e.g. it can be set to about 3 sec; the micturition threshold is a threshold set according to the functional status of a patient's bladder, and it can generally be set to about 20 cmH₂O; the judgement time is also the time set accroding to the functional status of a patient's bladder, e.g. it can be set to about 10 sec; the preset micturition duration is a time set accroding to the functional status of a patient's bladder, and it can generally be set to any value between 10 sec to 300 sec, or set individually as required by the patient. The above-mentioned micturition threshold is smaller than the threshold of releasing pressure, and the duration of releasing pressure is smaller the micturition duration; specifically, the method comprises: initially collecting the pressure in the bladder, judging whether the pressure is equal to or greater than the preset threshold of releasing pressure;
(1) if the judgement result is that the pressure is equal to or greater than the preset threshold of releasing pressure, then starting micturition to release pressure within the time to start releasing pressure, and the time for releasing pressure is the preset duration of releasing pressure;
(2) if the judgement result is that the pressure is smaller than the preset threshold of releasing pressure, then continuing to judge whether the pressure is equal to or greater than the micturition threshold, if the judgement result is that the pressure is equal to or greater than the preset micturition threshold, then further judging whether the duration of the pressure is equal to or greater than the judgement time, if the judgement result is that the duration of the pressure is equal to or greater than the judgement time, then performing micturition, and the time for micturition is the preset micturition duration.

Of course, it can also initially juge whether the pressure is equal to or greater than the preset micturition threshold;
(1) if the judgement result is that the pressure is equal to or greater to the preset micturition threshold, then further judging whether the pressure is equal to or greater than the preset threshold of releasing pressure, if the judgement result is that the pressure is equal to or greater than the preset threshold of releasing pressure, then starting micturition to release pressure within the time to start releasing pressure, and the time for releasing pressure is the preset duration of releasing pressure;
(2) if the judgement result is that the pressure is equal to or greater than the preset micturition threshold, then further judging whether the pressure is equal to or greater than the preset threshold of releasing pressure, if the judgement result is that the pressure is smaller than the preset threshold of releasing pressure, then continuing to judge whether the duration of the pressrue is equal to or greater than the judgement time, if the judgement result is that the duration of the pressure is equal to or greater than the judgement time, then performing micturition, and the time for micturition is the preset micturition duration;
(3) if the judgement result is that the pressure is smaller than the preset micturition threshold, then continuing to collect the pressure in the bladder for judgement.

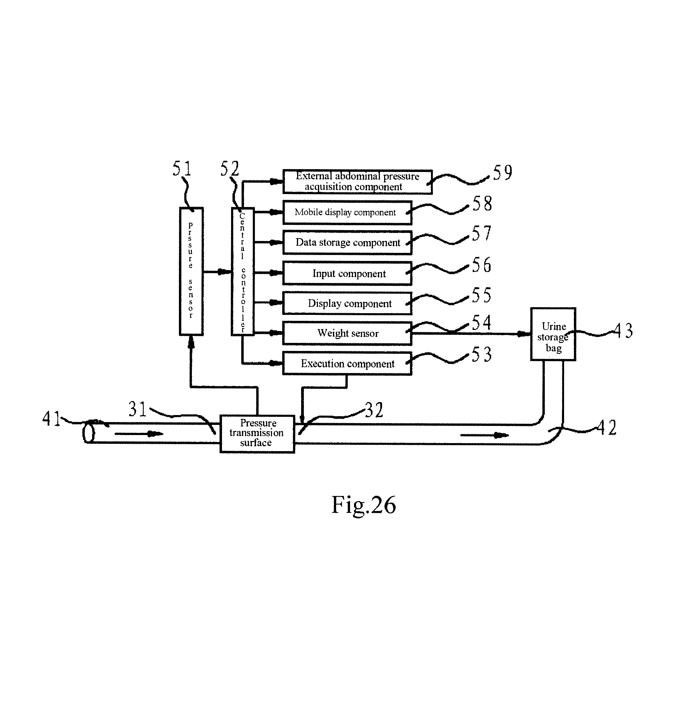

Figure 26 is a urine drainage control system for implementing the micturition method as shown in figures 24 and 25, the urine drainage control system comprises: a urine inlet 31 capable of connecting to the urinary catheter which is inserted into the human body and a urine outlet 32 capable of connecting to the urine storage bag 43 which stores urine, and a region located between the urine inlet 31 and the urine outlet 32 is provided with a pressure transmission surface, which pressure transmission surface can transmit the pressure in the bladder. The urine drainage control system further comprises:
a pressure sensor 51, which collects the bladder pressure via the above-mentioned pressure transmission surface, and generates and transmits a pressure signal;
a central controller 52, which receives the pressure signal outputted by the pressure sensor 51, and judges whether the pressure is equal to or greater than the threshold of releasing pressure, if the judgement result is yes, then starting micturition to release pressure within the time to start releasing pressure, and the time for releasing pressure is the preset duration of releasing pressure, if the judgement result is no, then continuing to judge whether the pressure is equal to or greater than the micturition threshold, if the judgement result is yes, then outputing an instruction of opening the urine outlet 32 to perform micturition, and the time for micturition is the preset micturition duration;
or the central controller 52 receives the pressure signals outputted by the pressure sensor 51, and judges whether the pressure is equal to or greater than the preset micturition threshold, if the judgement result is yes, then further judging whether the pressure is equal to or greater than the preset threshold of releasing pressure, if the judgement result is yes, then starting micturition to release pressure within the time to start releasing pressure, and the time for releasing pressure is the preset duration of releasing pressure; if the result for judging is no, then continuing to judge whether the duration of the pressure is equal to or greater than the judgement time, if the judgement result is yes, then outputing an instruction of opening the urine outlet 32 to perform micturition, the time for micturition is the preset micturition duration;
an execution component 53, which receives the instruction outputted by the central controller 52, and controls whether the urine flows out from the urine outlet 32.

The driving mechanism of the execution component 53 is an air pump, the execution component 53 further comprises an air sac, the central controller 52 controls the air pump to inflate the air sac according to the pressure signal from the pressure sensor 51, so that the air sac applies an external force acted on the urine outlet 32 to close the urine outlet 32; or controls to deflate the air sac, so as to remove the external force acted on the urine outlet 32 to open the urine outlet 32.

The urine drainage control system further comprises a weight sensor 54, a display component 55, an input component 56, a data storage component 57, a mobile display component 58 and an external abdominal pressure acquisition component 59. The weight sensor 54 is used for real-time monitoring the weight of urine in the urine storage 43, and transports the weight signal to the central controller 52. When the urine weight exceeds the preset value, the central controller 52 can send out a warning signal to change the urine storage bag 43, and the central controller 52 is capable of plotting a series of curves reflecting the bladder functions, such as urine flow rate curve, urinary output and weight change curve, according to the parameters such as weight signal, micturition time and urine density. The display component 55 is connected to the central controller 52, for displaying the monitored data and the artificially set data as well as the calculated results of the central controller 52; the input component 56 is connected to the central controller 52, for setting parameter and/or querying the stored record of the data storage component 57 and the calculated result of the central controller 52; the data stroage component 57 is used for the output of data and the input of data, the data storage component 57 is capable of storing the parameter set by the input component 56, as well as the urine flow rate curve, the urinary output curve, the bladder pressure curve and the abdominal pressure curve that are anlyzed and generated by the central controller, and is capable of transporting the above-mentioned curves to an external device, and is capable of further tranporting the parameter recorded on the external devices to the central controller; the external abdominal pressure acquisition component 59 can collect the abdominal pressure in the human body, and transports the collected signal to the central controller 52; the mobile display component 58 is wirelessly connected to the central controllor, for displaying the contents displayed by the external abdominal pressure acquisition component 59. This system can be powered by a battery or powered by a fixed power source.

The pressure transmission surface of the urine drainage control system is made of an elastic material, and is capable of deforming along with the change of pressure in the bladder. The urine drainage control system further comprises a control portion, the control portion comrpises a pressure transmission component 11 and a closure component 12, the pressure transmission component 11 is capable of contacting with the pressure transmission surface, and shifts in position by the action of force produced by the deformation of the pressure transmission surface; the closure component 12 is capable of shifting along with the shift in position of the pressure transmission component 11, when shifting in position, the closure component 12 is capable of controling whether the urine flows out from the urine outlet 32.

The urine drainge device, the urine drainage control device, the micturition method which protects the bladder function and the urine drainage control system provided by the present invention are all described above in detail. Particular examples are utilized herein to illustrate the principles and embodiments of the present invention, however, the examples described above are used for helping understanding the method of the present invention and the core idea thereof. It should be pointed out that an ordinary person skilled in the art can further performing various improvements and modifications to the present invention without departing from the principles of the present invention, and these improvements and modifications also fall within the protection scope of the claims of the present invention.

## Claims

1. A urine drainage device, comprising a urine inlet (31) capable of connecting to a urinary catheter which is inserted into human body and a urine outlet (32) capable of connecting to a urine storage bag (43) which stores urine, **characterized in that**
the urine drainge device further comprises a bionic bladder unit (2), the bionic bladder unit (2) comprises a main body component (21) capable of forming a urine storage cavity (215) which stores urine, the urine storage cavity (215) is communicated with the urine inlet (31) and the urine outlet (32) via a connecting tube (216), the main body component (21) comprises a first pressure transmission surface (211) and a second pressure transmission surface (213) capable of deforming with the change of the urine presure in the urine storage cavity (215), the connecting tube (216) communicating the urine storage cavity (215) with the urine outlet (32) is a lower connecting tube (216a), the lower connecting tube (216a) is provided with a first action area (212) and a second action area (214) on which an external force can be applied, the first action area (212) and the second action area (214) are oppositely disposed;
when the external force is applied on both the first action area (212) and the second action area (214) simultaneously, the communication between the urine storage cavity (215) and the urine outlet (32) is blocked; according to the deformation of the first pressure transmission surface (211) and/or the second pressure transmission surface (213), the external force applied on the first action area (212) and/or the second action area (214) can be removed, and when any one external force applied on the first action area (212) and the second action area (214) is removed, the blockage between the urine storage cavity (215) and the urine outlet (32) can be removed, the communication between the urine storage cavity (215) and the urine outlet (32) can be restored.

2. The urine drainage device according to claim 1, **characterized in that** the main body component (21) comprises two diaphragms, one diaphragm forms the first pressure transmission surface (211) and the first action area (212), and the other diaphragm forms the second pressure transmission surface (213) and the second action area (214), the two diaphragms are fastened and sealed, and the internal cavity formed by the oppositely disposed first pressure transmission surface (211) and second pressure transmission surface (213) is the urine storage cavity (215).

3. The urine drainage device according to claim 1, **characterized in that** the bionic bladder unit (2) further comprises a hard cover housing (22), and after fastening, the cover housing (22) is capable of covering the portions on the main body component (21) except the first pressure transmission surface (211), the first action area (212), the second pressure transmission surface (213) and the second action area (214).

4. The urine drainage device according to claim 1, **characterized in that** the urine inlet (31) and the urine outlet (32) are connected to a urine inlet tube (41) and a urine outlet tube (42) respectively, and the urine inlet tube (41) is provided with a urinary catheter connector (44) for connecting to the urinary catheter.

5. The urine drainage device according to claim 4, **characterized in that** the urinary catheter connector (44) and/or the urine inlet tube (41) are provided with a self-sealable region for temperature collecting (45) and/or urine sampling (46).

6. A urine drainage control device, comprising a urine drainage portion, a control portion and a housing (6) containing the urine drainage portion and the control portion, **characterized in that** the urine drainage portion is the urine drainage device according to any of claims 1-5; the control portion comprises a pressure transmission component (11) and a closure component (12), the pressure transmission component (11) is capable of contacting with the deformed first pressure transmission surface (211) and/or second pressure transmission surface (213), and shifts in position by the action of the force produced by the deformation of the first pressure transmission surface (211) and/or the second pressue transmitting surface (213); the closure component (12) is capable of shifting along with the shift in position of the pressure transmission component (11), and when shifting in position, the closure component (12) is capable of applying or removing the external force acted on the first action area (212) and/or the second action area (214), thereby blocking or unblocking the communication between the urine storage cavity (215) and the urine outlet (32).

7. The urine drainage control device according to claim 6, **characterized in that** there is a gap between the pressure transmission component (11) and the corresponding pressure transmission surface thereof, and when the pressure transmission surface deforms along with the increasing pressure in the urine storage cavity (215), the gap is tapered until eliminated.

8. The urine drainage control device according to claim 6, **characterized in that** the control portion further comprises a resistance component (13), the resistance component (13) applies a predetermined resistance on the pressure transmission component (11) and/or the closure component (12) to block the deformation of the corresponding pressure transmission surface, and when the force of the corresponding pressure transmission surface acted on the pressure transmission component (11) or the closure component (12) is greater than the predetermined resistance, the pressure transmission component (11) drives the closure component (12) to shift in position.

9. The urine drainage control device according to claim 8, **characterized in that** the resistance of the resistance component (13) is produced by a spring (131), one end of the spring (131) connects to the housing (6), and the other end connects to the pressure transmission component (11) or the closure component (12).

10. The urine drainage control device according to claim 9, **characterized in that** the pressure transmission component (11) and the closure component (12) are a unitary structure; the control portion further comprises a rotation axis (15), and when the pressure transmission component (11) shifts in position along with the deformation of the corresponding pressure transmission surface, the pressure transmission component (11) and the closure component (12) rotate together around the rotation axis (15), so that the closure component (12) applies or removes the external force on the corresponding action area, thereby blocking or unblocking the communication between the urine storage cavity (215) and the urine outlet (32).

11. The urine drainage control device according to claim 9, **characterized in that** the pressure transmission component (11) and the closure component (12) are a unitary structure; the pressure transmission component (11) is provided with a guide rod, the housing (6) is provided with a socket, the guide rod is capable of shifting in the socket in the stretching direction of the spring (131), and when the pressure transmission component (11) shifts in position along with the deformation of the corresponding pressure transmission surface, the pressure transmission component (11) and the closure component (12) shift together along with the guide rod in the direction of the socket, so that the closure component (12) applies or removes the external force on the corresponding action area, thereby blocking or unblocking the communication between the urine storage cavity (215)and the urine outlet (32).

12. The urine drainage control device according to claim 9, **characterized in that** the pressure transmission component (11) comprises a first gear (111), the closure component (12) comprises a closure head (121) and a second gear (122) engaged with the first gear (111), both the first gear (111) and the second gear (122) are fixed with respect to the housing (6); the spring (131) and the pressure transmission component (11) are connected by a movable axis, and when the pressure transmission component (11) shifts in position along with the deformation of the corresponding pressure transmission surface, the first gear (111) drives the rotation of the second gear (122) so as to drive the closure head (121) to apply or remove the external force on the corresponding action area, thereby blocking or unblocking the communication between the urine storage cavity (215) and the urine outlet (32).

13. The urine drainage control device according to claim 9, **characterized in that** the pressure transmission component (11) and the closure component (12) are hinged, and when the pressure transmission component (11) shifts in position along with the deformation of the corresponding pressure transmission surface, it drives the rotation of the closure component (12), applies or removes the external force on the corresponding action area, thereby blocking or unblocking the communication between the urine storage cavity (215) and the urine outlet (32).

14. The urine drainage control device according to any of claims 6-13, **characterized in that** the control portion further comprises a manually controlled valve (7), so as to manually apply or remove the external force on the corresponding action area, thereby blocking or unblocking the communication between the urine storage cavity (215) and the urine outlet (32).

15. A urine drainage control system, comprising a urine drainage control portion and an electric control portion, the urine drainage control portion and the electric control portion are contained in the housing (6), **characterized in that** the urine drainage control portion is the urine drainage control device according to any of claims 6-13;
the electric control portion comprises a central controller (52), an execution component (53), and a pressure sensor (51) that collects the pressure in the bladder, the central controller (52) receives the pressure signal outputted by the pressure sensor (51), and when the pressure is greater than the micturition threshold and the duration is equal to or greater than the preset judgement time, the central controller (52) outputs an instruction of communicating the urine storage cavity (215) with the urine outlet (32) to the execution componet (53).

16. The urine drainage control system according to claim 15, **characterized in that** the pressure sensor (51) collects the pressue transmitted by the corresponding pressure transmission surface, and the execution component (53) applies or removes the external force on the corresponding action aera, thereby blocking or unblocking the communication between the urine storage cavity (215) and urine outlet (32).

17. The urine drainage control system according to claim 15, **characterized in that** the driving mechanism of the execution component (53) comprises an electric motor, a solenoid valve and an air pump.

18. The urine drainage control system according to claim 17, **characterized in that** the execution component (53) further comprises an air sac, the central controller (52) controls the air pump to inflate the air sac according to the pressure signal from the pressure ssensor (51) in order that the air sac applies external force acted on the corresponding action area, or controls the air sac to deflate in order to remove the external force acted on the corresponding action area.

19. The urine drainage control system according to claim 15, **characterized in that** the system further comprises a weight sensor (54), the weight sensor (54) is used for real-time monitoring the weight of urine in the urine storage bag (43), and outputs weight signal to the central controller (52).

20. The urine drainage control system according to claim 15, **characterized in that** the system further comprises a display component (55), an input component (56), and a data storage component (57);
the display component (55) connects to the central controllor (52), for displaying the monitored data and the artificially set data as well as the calculated result by the central controller (52);
the input component (56) connects to the central controller (52), for setting parameter and/or querying the storage record of the data storage component (57) and the calculated result of the central controller (52);
the data strorage component (57) is used for the output of data and the input of data, the data strorage component (57) is capable of storing the parameter set by the input component (56), as well as the urine flow rate curve, the urinary output curve, the bladder pressure curve and the abdominal pressure curve that are anlyzed and generated by the central controller (52), and is capable of movably transporting the above-mentioned curves to an external device, and is capable of tranporting the parameter recorded on the external device to the central controller (52).

21. A micturition method which protects the bladder function, **characterized in that** the method comprises:
presetting a threshold of releasing pressure as well as a time to start releasing pressure and a duration of releasing pressure;
collecting the pressure in the bladder;
judging whether the pressure is equal to or greater than the preset threshold of releasing pressure;
if the judgement result is that the pressure is equal to or greater than the preset threshold of releasing pressure, then starts micturition to release pressure within the time to start releasing pressure, and the time for releasing pressure is the preset duration of releasing pressure.

22. The micturition method according to claim 21, **characterized in that** the method further comprises:
presetting a micturition threshold as well as a judgement time and a micturition duration, the micturition threshold is smaller than the above-mentioned threshold of releasing pressure;
collecting the pressure in the bladder;
judging whether the pressure is equal to or greater than the preset threshold of releasing pressure;
(1) if the judgement result is that the pressure is equal to or greater than the preset threshold of releasing pressure, then stating micturition to release pressure within the time to start releasing pressure, and the time for releasing pressure is the preset duration of releasing pressure;
(2) if the judgement result is that the pressure is smaller than the preset threshold of releasing pressure, then continuing to judge whether the pressure is equal to or greater than the micturition threshold, if the judgement result is that the pressure is equal to or greater than the preset micturition threshold, then further judging whether the duration of the pressure is equal to or greater than the judgement time, if the judgement result is that the duration of the pressure is equal to or greater than the judgement time, then performing micturition, and the time for micturition is the preset micturition duration;
or judging whether the pressure is equal to or greater than the preset micturition threshold;
(1) if the judgement result is that the pressure is equal to or greater than the preset micturition threshold, then further judging whether the pressure is equal to or greater than the preset threshold of releasing pressure, if the judgement result is that the pressrue is equal to or greater than the preset threshold of releasing pressure, then starting the micturition to release pressure within the time to start releasing pressure, and the time for releasing pressure is the preset duration of releasing pressure;
(2) if the judgement result is that the pressure is equal to or greater than the micturition threshold and smaller than the threshold of releasing pressure, then continuing to judge whether the duration of the pressure is equal to or greater than the judgement time, if the judgement result is that the duration of the pressure is equal to or greater than the judgement time, then performing micturition, and the time for micturition is the preset micturition duration;
(3) if the judgement result is that the pressure is smaller than the preset micturition threshold, then continuing to collect the pressure in the bladder for performing judgement.

23. The micturition method according to claim 22, **characterized in that** the time to start releasing pressure is smaller than the judgement time.

24. The micturition method according to claim 22, **characterized in that** the duration of releasing pressure is smaller than the micturition duration.

25. A urine drainage control system, comprising a urine inlet (31) capable of connecting to a urinary catheter which is inserted into human body and a urine outlet (32) capable of connecting to a urine storage bag (43) which stores urine, as well as at least a pressure transmission surface on at least one area between the urine inlet (31) and the urine outlet (32), the pressure transmission surface is capable of transmitting the pressure in the bladder, **characterized in that** the urine drainage control system further comprises:
a pressure sensor (51), the pressure sensor (51) collects the pressure in the bladder through the pressure transmission surface, and generates and transports a pressure signal;
a central contoller (52), the central contoller (52) receives the pressure signal outputted by the pressure sensor (51), and judges whether the pressure is equal to or greater than the threshold of releasing pressure, if the judgement result is yes, then starting the micturition to release pressure within the time to start releasing pressure, and the time for releasing pressure is the preset duration of releasing pressure, if the judgement result is no, then continuing to judge whether the pressure is equal to or greater than the micturition threshold, if the judgement result is yes, then further judging whether the duration of the pressure is equal to or greater than the judgement time, if the judgement result is yes, then outputing an instruction of openning the urine outlet (32) to perform micturition, and the time for micturition is the preset duration of micturition;
or the central controller (52) recives the pressure signal outputted by the pressure sensor (51), and judges whether the pressure is equal to or greater than the preset micturition threshold, if the judgement result is yes, then further judging whether the pressure is equal to or greater than the preset threshold of releasing pressure, if the judgement result is yes, then starting the micturition to release pressrue within the time to start releasing pressure; if the judgement result is no, then continuing judging whether the duration of the pressure is equal to or greater than the judgement time, if the judgement result is yes, then outputing an instruction of opening the urine outlet (32) to perform micturition, and the time for micturition is the preset micturition duration;
an execution component (53), which receives the instruction from the central controller (52), and controls whether the urine flows out from the urine outlet (32).

26. The urine drainage control system according to claim 25, **characterized in that** the driving mechanism of the execution component (53) comprises an electric motor, a solenoid valve and an air pump.

27. The urine drainage control system according to claim 26, **characterized in that** the execution component (53) further comprises an air sac, the central controller (52) controls the air pump to inflate the air sac according to the pressure signal from the pressure sensor (51) in order that the air sac applies an external force acted on the urine outlet (32) to close the urine outlet (32); or controls to deflate the air sac in order to remove the external force acted on the urine outlet (32) to open the urine outlet (32).

28. The urine drainage control system according to claim 25, **characterized in that** the system further comprises a weight sensor (54), the weight sensor (54) is used for real-time monitoring the weight of urine in the urine storage (43), and transports the weight signal to the central controller (52).

29. The urine drainage control system according to claim 25, **characterized in that** the system further comprises a display component (55), an input component (56), and a data storage component (57);
the display component (55) is connected to the central controller (52), for displaying the monitored data and the artificially set data as well as the calculated result of the central controller (52);
the input component (56) is connected to the central controller (52), for setting parameter and/or querying the storage record of the data storage component (57) and the calculated result of the central controller (52);
the data stroage component (57) is used for the output of data and the input of data, the data storage component (57) is capable of storing the parameter set by the input component (56), as well as the urine flow rate curve, the urinary output curve, the bladder pressure curve and the abdominal pressure curve that are anlyzed and generated by the central controller (52), and is capable of movably transporting the above-mentioned curves to an external device, and is capable of tranporting the parameter recorded on the external device to the central controller (52).

30. The urine drainage control system according to claim 25, **characterized in that** the pressure transmission surface is made of an elastic material, and is capable of deforming along with the change of pressure in the bladder.

31. The urine drainage control system according to claim 30, **characterized in that** the urine drainage control system further comprises a control portion, the control portion comrpises a pressure transmission component (11) and a closure component (12), the pressure transmission component (11) is capable of contacting with the pressure transmission surface and shifts in position by the action of force produced by the deformation of the pressure transmission surface; the closure component (12) is capable of shifting along with the shift in position of the pressure transmission component (11), and when shifting in position, the closure component (12) is capable of controling whether the urine flows out from the urine outlet (32).
